(19) Europäisches Patentamt
European Patent Office
Office européen des brevets

(11) Veröffentlichungsnummer: **0 438 563 B1**

(12) # EUROPÄISCHE PATENTSCHRIFT

(45) Veröffentlichungstag der Patentschrift: **07.12.94**

(51) Int. Cl.5: **C07C 25/13**, C09K 19/30, C07C 255/50, C07C 43/225

(21) Anmeldenummer: **90912038.8**

(22) Anmeldetag: **13.08.90**

(86) Internationale Anmeldenummer: **PCT/EP90/01330**

(87) Internationale Veröffentlichungsnummer: **WO 91/02709 (07.03.91 91/06)**

Die Akte enthält technische Angaben, die nach dem Eingang der Anmeldung eingereicht wurden und die nicht in dieser Patentschrift enthalten sind.

(54) **PHENYLCYCLOHEXANE UND FLÜSSIGKRISTALLINES MEDIUM.**

(30) Priorität: **16.08.89 DE 3926871**
**01.03.90 DE 4006313**
**27.04.90 DE 4013467**
**25.04.90 DE 4013084**
**03.07.90 DE 4021154**

(43) Veröffentlichungstag der Anmeldung: **31.07.91 Patentblatt 91/31**

(45) Bekanntmachung des Hinweises auf die Patenterteilung: **07.12.94 Patentblatt 94/49**

(84) Benannte Vertragsstaaten: **AT BE CH DE DK ES FR GB IT LI LU NL SE**

(56) Entgegenhaltungen:
**EP-A- 0 167 912**
**EP-A- 0 315 014**

(73) Patentinhaber: **MERCK PATENT GmbH**
**Postfach,**
**Frankfurter Strasse 250**
**D-64271 Darmstadt (DE)**

(72) Erfinder: **WÄCHTLER, Andreas**
**Goethestrasse 34**
**D-6103 Griesheim (DE)**
Erfinder: **HITTICH, Reinhard**
**Am Kirchberg 11**
**D-6101 Modautal 1 (DE)**
Erfinder: **POETSCH, Eike**
**Am Buchwald 4**
**D-6109 Mühltal 6 (DE)**
Erfinder: **PLACH, Herbert**
**Wingertsbergstrasse 5**
**D-6100 Darmstadt (DE)**
Erfinder: **COATES, David**
**87 Sopwith Crescent**
**Merley**
**Wimborne Dorset BH21 3SW (GB)**

**EP 0 438 563 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **RIEGER, Bernhard Wacore-Tamaga-wagakuen**
**2834 Ootadaira, Nara-machi**
**Midori-ku, Yokohama-shi**
**Kanagawa Pref. 227 (DE)**
Erfinder: **KRAUSE, Joachim**
**Samuel-Morse-Strasse 14**
**D-6110 Dieburg (DE)**

## Beschreibung

Die Erfindung betrifft flüssigkristalline Medien für elektrooptische Anzeigen mit mindestens zwei flüssigkristallinen Komponenten, wobei mindestens eine Komponente ein Phenylcyclohexan der Formel I ist,

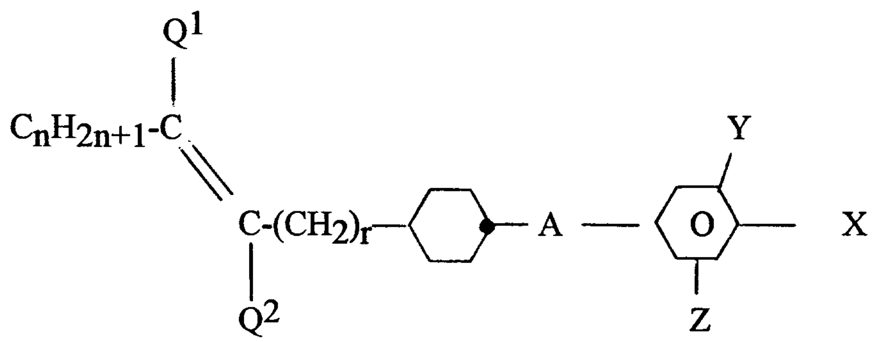

worin n 0 bis 7, $Q^1$ und $Q^2$ H, r 0, 1 oder 2, A trans-1,4-Cyclohexylen, 1,4-Phenylen, 3-Fluor-1,4-phenylen oder eine Einfachbindung, X F, Cl, $-CF_3$, -CN, $-OCF_3$ oder $-OCHF_2$, und Y und Z jeweils unabhängig voneinander H oder F bedeuten, mit der Maßgabe, daß im Falle X = CN Y und/oder Z F bedeuten sowie Phenylcyclohexane der Formel I, worin Y = F und Z = H oder F, r = 0, X = $-CF_3$, $-OCF_3$ oder $-OCHF_2$ oder X = CN, Z = F und Y = H oder F bedeutet, und es als weiteren Bestandteil eine Verbindung der Formel 5 enthält,

R'-L-C≡C-E-R"      5

worin L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc-sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, und

R' und R" jeweils unabhängig voneinander Alkyl, Alkenyl, Alkoxy, Alkenyloxy oder Alkanoyloxy mit bis zu 8 Kohlenstoffatomen,

oder R" auch -CN, $-CF_3$, $-OCF_3$, $-OCHF_2$, F, Cl oder -NCS ist.

Aus der EP-OS 0 122 389 sind ähnliche Verbindungen, beispielsweise der Formel A

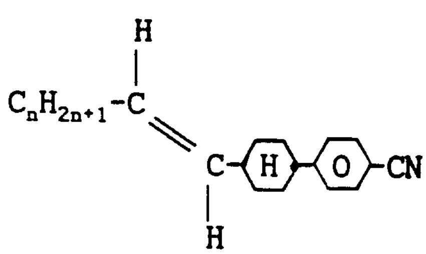

A

bekannt. Diese Verbindungen werden jedoch nicht allen Anforderungen, insbesondere hinsichtlich (Langzeit)-Stabilität, beispielsweise für Anwendungen in Displays mit aktiver Matrix, gerecht.

Aus der DE-OS 29 07 332 sind ähnliche Verbindungen der Formel

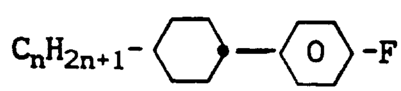

bekannt. Diese nematogenen Verbindungen eignen sich vorzüglich zur Verbesserung des Tieftemperaturverhaltens nematischer Gemische, zeigen jedoch andererseits relativ hohe Werte für den Dampfdruck für kleine Werte von n.

Aus der EP-OS 02 80 902 sind ähnliche Verbindungen der Formel

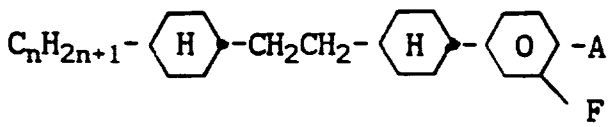

bekannt, worin A F, Cl, Br, H oder CN bedeutet. Diese Verbindungen haben zwar niedrige Werte für den Dampfdruck, jedoch andererseits deutlich smektogene Eigenschaften. Es besteht somit ein Bedarf an hoch nematogenen Verbindungen mit geringen Werten für den Dampfdruck.

Aus der EP-A 167 912 sind ähnliche Verbindungen mit einer 4-Alkenyl- oder 2Z-Alkenyl-Seitenkette bekannt. In der EP-A 315 014 werden flüssigkristalline Medien beschreiben enthaltend ähnliche Verbindungen mit einer 4-Alkenyl-Seitenkette. Es gibt jedoch keine Hinweise, daß die Verwendung von Medien enthaltend 1E-, 2E- oder 3E-Alkenyl-Reste zu LCDs mit verbesserten Eigenschaften führt.

Insbesondere bei Anzeigen vom Supertwisttyp (STN) mit Verdrillungswinkeln von deutlich mehr als 220 ° oder bei Anzeigen mit aktiver Matrix weisen die bisher eingesetzten Materialien Nachteile auf.

Diese Verbindungen der Formel I können wie ähnliche, z.B. aus den DE-OS 26 36 684 und 29 07 332 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtlich gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung, ungünstige dielektrische und/oder elastische Eigenschaften.

Der Erfindung lag die Aufgabe zugrunde, neue flüssigkristalline Verbindungen aufzufinden, die als Komponente flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu tiefen Temperaturen, hervorragender chemischer Stabilität, ausgeprägtem $\epsilon_L$ bei positiver dielektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität und hervorragenden elastischen Eigenschaften. Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophase in einem für die elektrooptische Verwendung günstig gelegenen Temperaturbereich.

Gegenstand der Erfindung sind somit flüssigkristalline Medien der genannten Art, Verbindungen der Forme I, worin Y = F, Z = H oder F und r = O, Verbindungen der Formel I, worin r = O und X = Cl, $-OCF_3$ oder $-OCHF_2$, Verbindungen der Formel I, worin

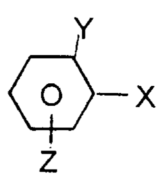

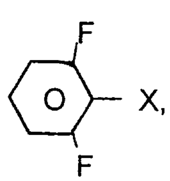

Verbindungen der Formel I, worin X $-CF_3$, $-OCF_3$ oder-$OCHF_2$ sowie Verbindungen der Formel I, worin r = 2 oder 3, A = 1,4-Phenylen, 3-Fluor-1,4-phenylen oder eine Einfachbindung, X = F oder Cl, Z = H und Y = F oder H bedeutet, sowie die Verwendung dieser Verbindungen als Komponenten flüssigkristalliner Medien, sowie elektrooptische Anzeigen, die derartige Medien enthalten.

Gegenstand der Erfindung ist ferner ein Verfahren zur Herstellung von Verbindungen der Forme I, worin r = O, Y = F und Z = H oder F bedeutet, ein Verfahren zur Herstellung von dafür verwendeten Phosphoniumsalzen sowie neue Zwischenprodukte dieser Verfahren.

In Verbindungen der Formel I sind die Alkylgruppen $C_nH_{2n+1}$ vorzugsweise geradkettig. Dementsprechend bedeutet $C_nH_{2n+1}$ vorzugsweise Methyl, Ethyl, n-Propyl, n-Butyl, n-Pentyl, n-Hexyl, n-Heptyl oder ebenfalls bevorzugt H. n ist vorzugsweise 0, 1, 2, 3, 4 oder 5.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl), 2-Methyl-butyl, Isopentyl (= 3-Methylbutyl), 2-Methylpentyl, 3-Methylpentyl, 2-Heptyl (= 1-Methylhexyl), 2-Octyl (=1-Methylheptyl), 2-Ethylhexyl.

Der Rest

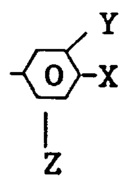

ist vorzugsweise

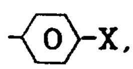 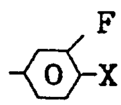 oder 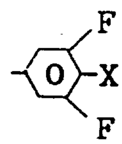

X ist vorzugsweise F, Cl, $-CF_3$, $OCHF_2$ oder $-OCF_3$. Ferner bevorzugt ist X = CN, insbesondere wenn gleichzeitig Z = F und Y = F oder H, insbesondere Y = F bedeutet. Ganz besonders bevorzugt sind die Verbindungen mit X = $CF_3$, $-OCF_3$ oder $-OCHF_2$.

r ist vorzugsweise 0. A ist vorzugsweise eine Einfachbindung und ferner bevorzugt trans-1,4-Cyclohexylen oder 1,4-Phenylen. Im Falle r = 2, Z = H, X = F oder Cl und gleichzeitig Y = F oder H ist A vorzugsweise die Einfachbindung, 1,4-Phenylen oder 3-Fluor-1,4-phenylen.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise wird ein Aldehyd der Formel II

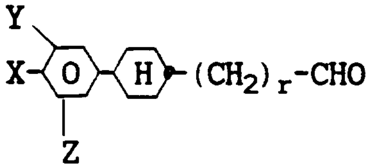

II

worin X, Y und Z die angegebene Bedeutung haben, mit einem entsprechenden Phosphoniumsalz nach Wittig in Verbindungen der Formel I übergeführt.

Die Ausgangsstoffe sowie ihre reaktionsfähigen Derivate sind zum Teil bekannt, zum Teil können sie ohne Schwierigkeiten nach Standardverfahren der organischen Chemie aus literaturbekannten Verbindungen

hergestellt werden. Die zur Synthese geeigneten Vorstufen der Formel II sind beispielsweise nach folgendem Syntheseschema erhältlich:

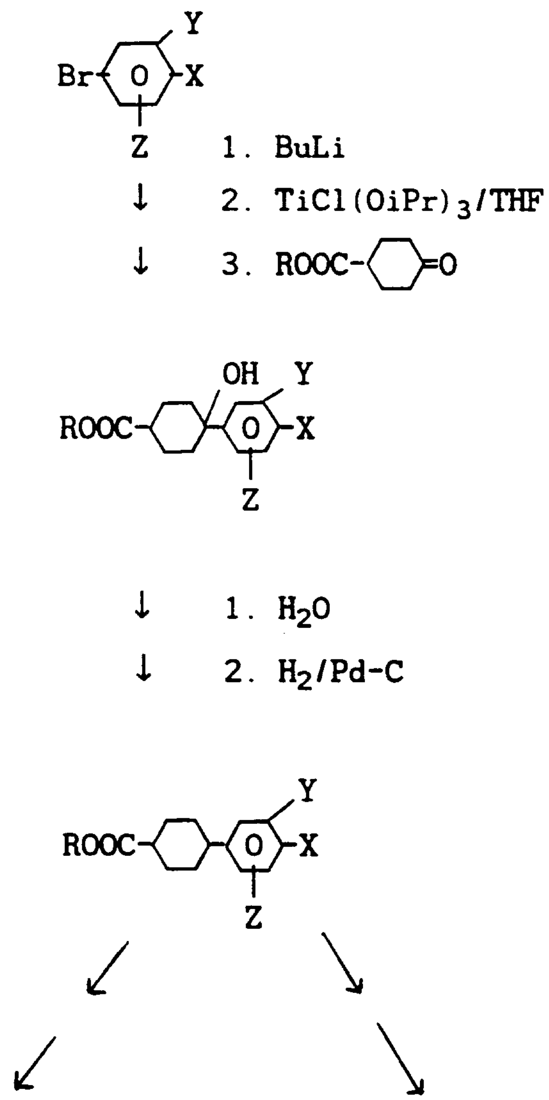

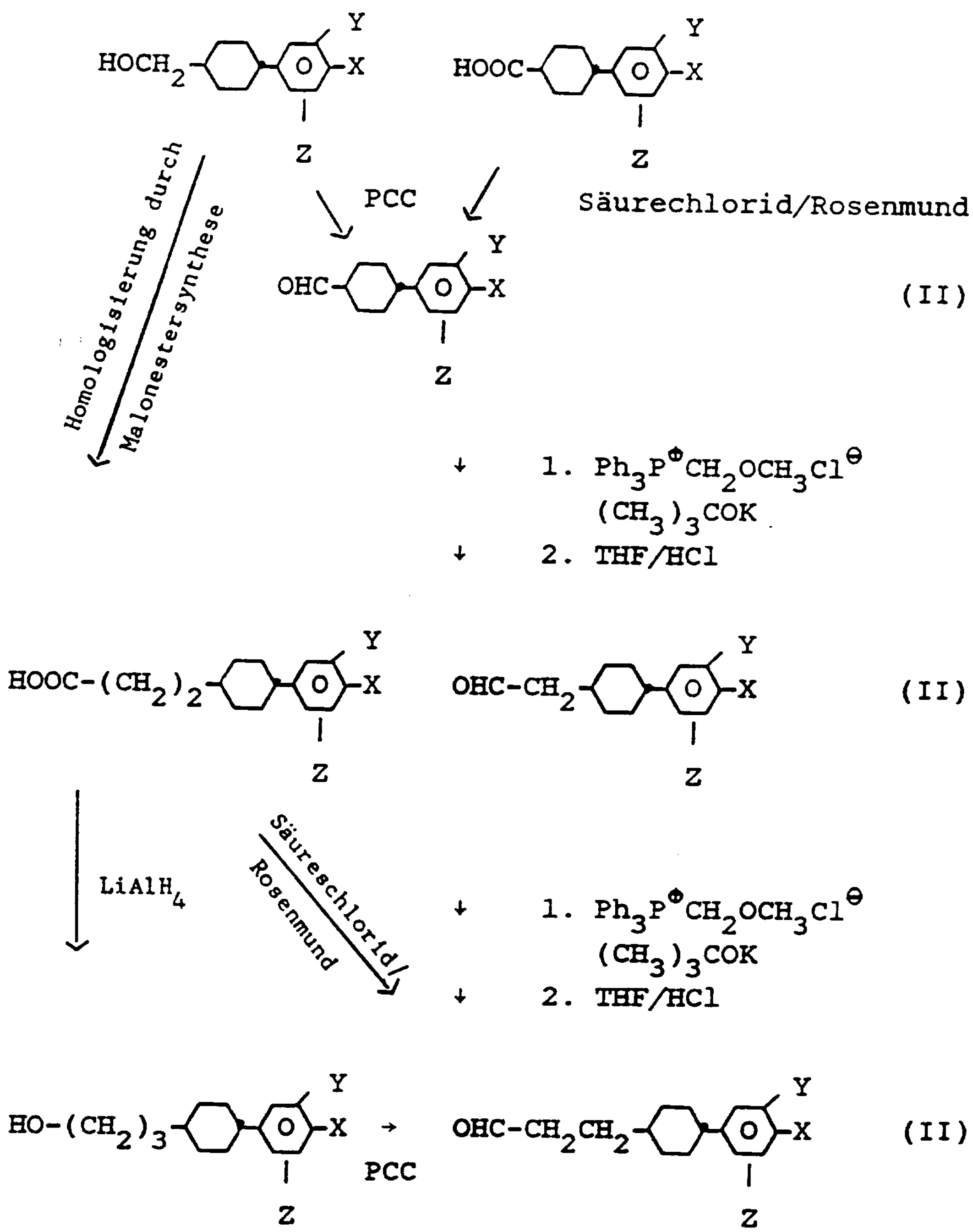


Die aus dem entsprechenden Brombenzolderivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung erhält man nach üblichen Methoden den trans-Cyclohexancarbonsäureester. Aus letzterem erhält man nach üblichem Standardverfahren die geeigneten Vorprodukte der Formel II.

Eine Synthesemöglichkeit für Verbindungen mit A = trans-1,4-Cyclohexylen oder 1,4-Phenylen ist folgendem Schema zu entnehmen:

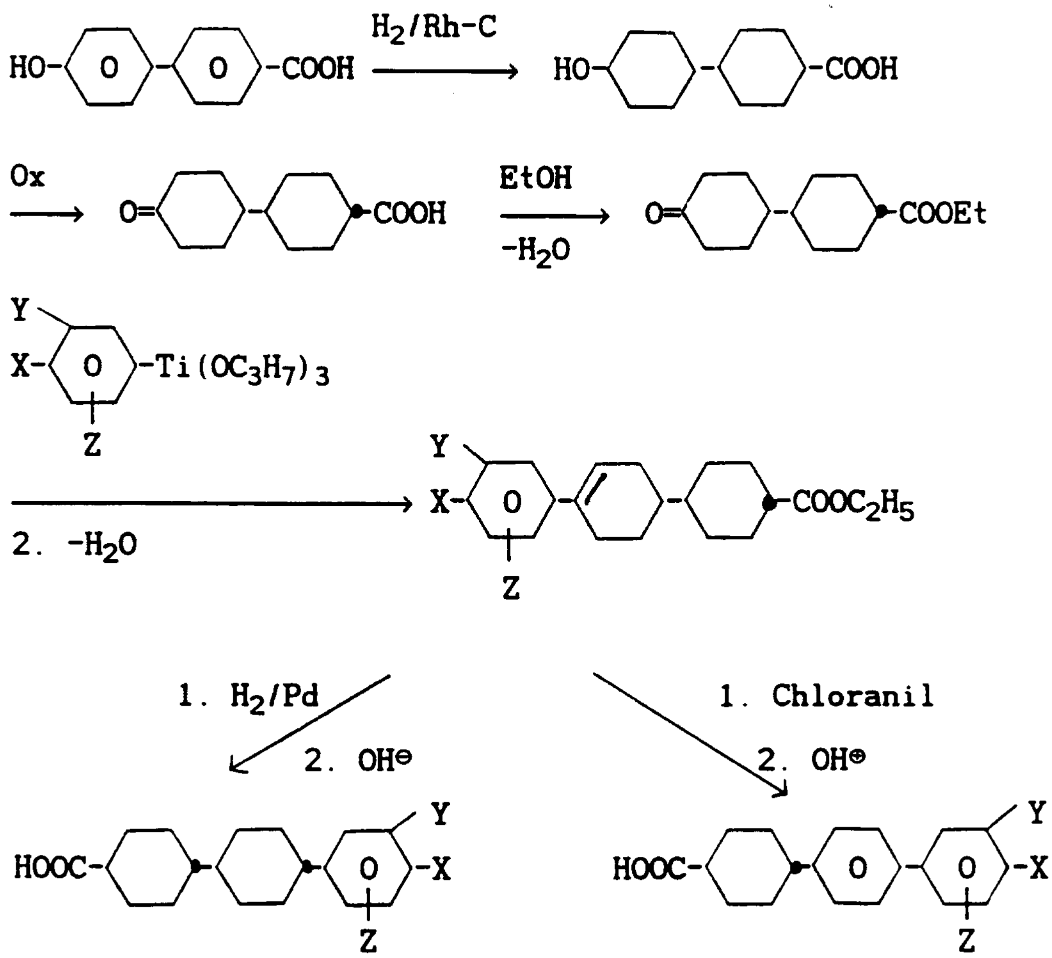


Durch Homologisierung der Cyclohexancarbonsäuren bzw. entsprechender Aldehyde erhält man die erfindungsgemäßen Verbindungen in völliger Analogie zu den weiter oben angegebenen Syntheseschemata.

Die Verbindungen der Formel I mit A = 3-Fluor-1,4-phenylen erhöht man in völliger Analogie zum ersten Syntheseschema (Herstellung von Verbindungen mit A = Einfachbindung) durch Einsatz von

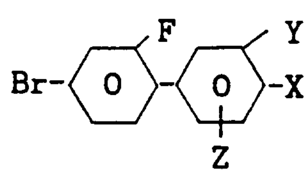

anstelle des Brombenzolderivates. Die Brombiphenylverbindung kann in an sich bekannter Weise durch übergangsmetallkatalysierte Kopplungsreaktionen (E. Poetsch, Kontakte (Darmstadt) 1988 (2), S. 15) hergestellt werden.

Es ist für den Fachmann offensichtlich, daß die oben genannten Syntheseverfahren auch dahingehend modifiziert werden können, daß die beschriebenen Synthesen mit Vorstufen durchgeführt werden, die anstelle des Restes X eine in X überführbare Gruppe tragen. Beispielsweise können Alkoxyverbindungen durch Etherspaltung in entsprechende Phenole umgewandelt werden, aus denen die $OCF_3$- und $OCF_2H$-Verbindungen nach Routinemethoden durch Umsetzung mit $CCl_4$/HF bzw. $CClF_2H$/NaOH hergestellt werden können. Aus den entsprechenden Benzoesäuren lassen sich die Nitrile oder durch Behandeln mit $SF_4$ die $CF_3$-Verbindungen herstellen.

Oft erweist es sich jedoch als vorteilhaft, ein Phosphoniumsalz der Formel III

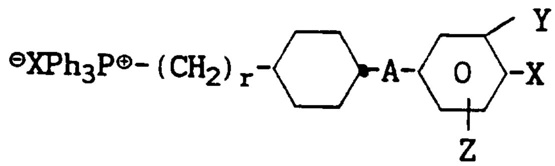

III

nach Wittig mit einem entsprechenden Aldehyd unzusetzen. Die Verbindungen der Formel III erhält man aus den oben beschriebenen Cyclohexylmethylalkoholen nach folgendem Schema:

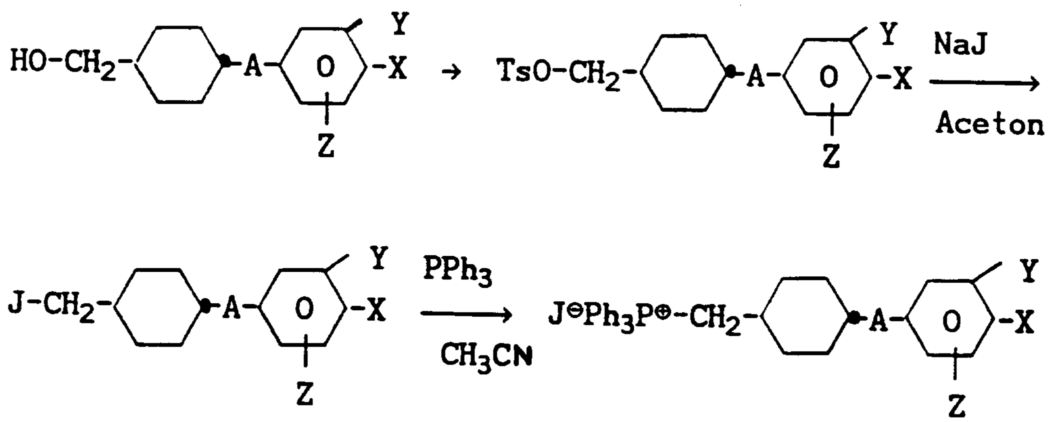


Durch Homologisierung erhält man die entsprechenden höheren Phosphoniumsalze:

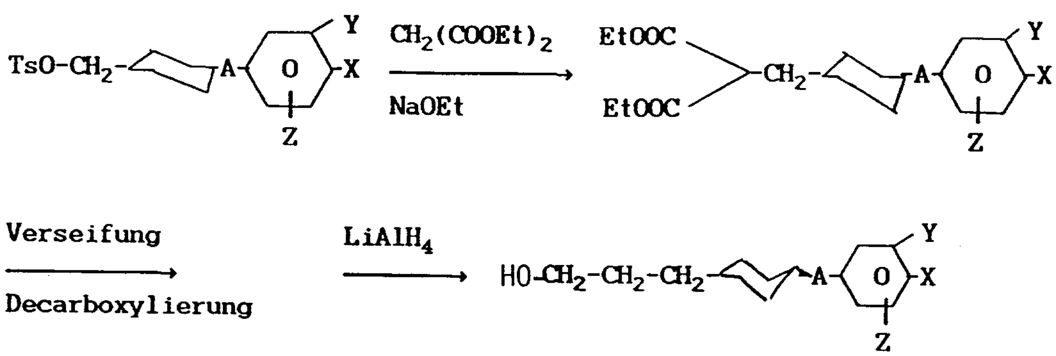


Die Homologisierung um ein C-Atom erfolgt am besten über die Nitrile:

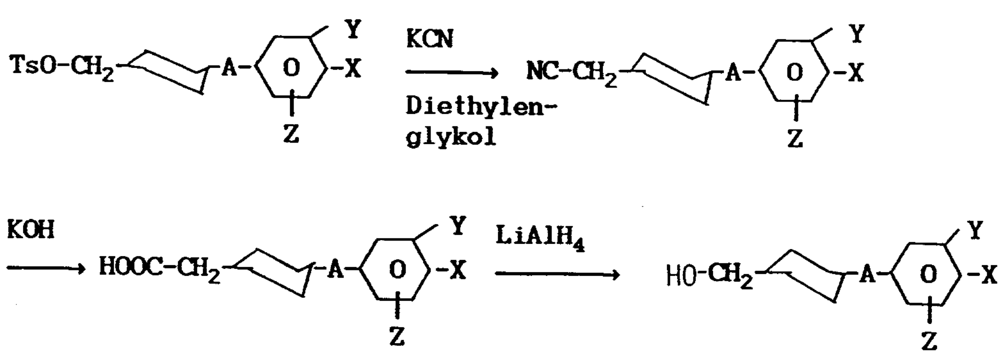

Bei basenempfindlichen Nitrilen erfolgt die Umwandlung zum Alkohol am besten durch Reduktion des Nitrils zum Aldehyd mit DIBAH und anschließend zum Alkohol mit $LiAlH_4$.

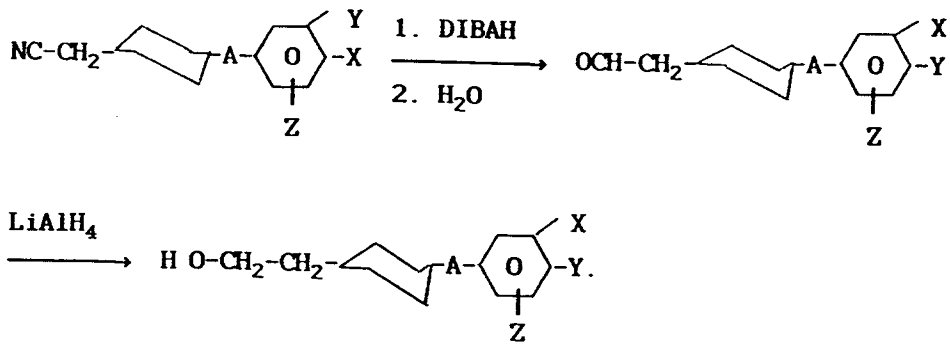


Aus den Alkoholen gewinnt man nach Finkelstein über die Tosylate die entsprechenden Jodide, die dann durch Kochen in Acetonitril mit $PPh_3$ in die entsprechenden Phosphoniumsalze überführt werden.

Die aus diesen Phosphoniumsalzen abgeleiteten Ylide (hergestellt unter Sauerstoffausschluß bei -70 °C durch Behandlung mit n-BuLi in Hexan) reagieren mit Aldehyden zu den cis-Olefinen. Diese cis-Olefinen werden dann nach E. Vedejs und P.C. Fuchs (JACS 95 822 (1973)) nach der Phosphor-Betain-Methode zu den trans-Olefinen isomerisiert.

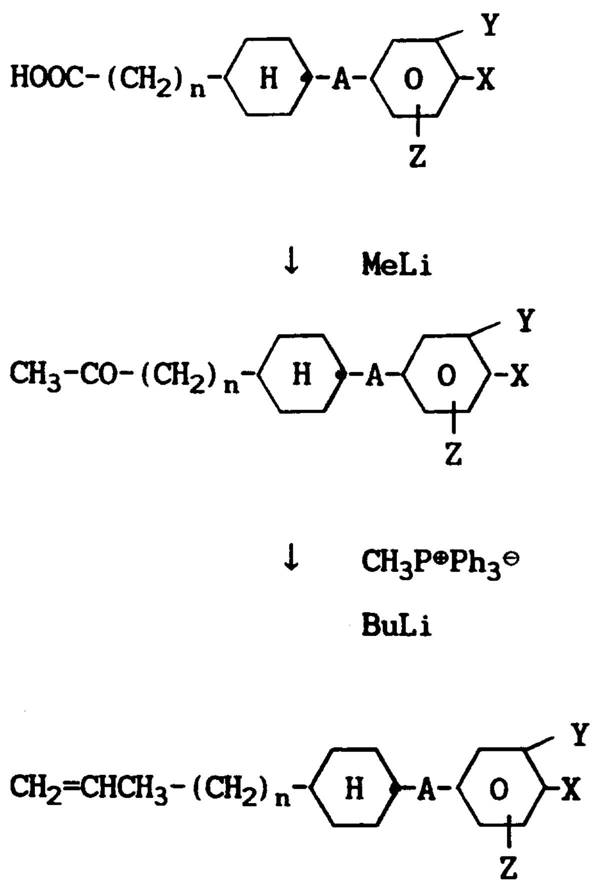


II'

III'

Die Ausgangsstoffe der Formel II' sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind Carbonsäuren der Formel II' nach folgenden Syntheseschemata erhältlich sind:

Schema 1:

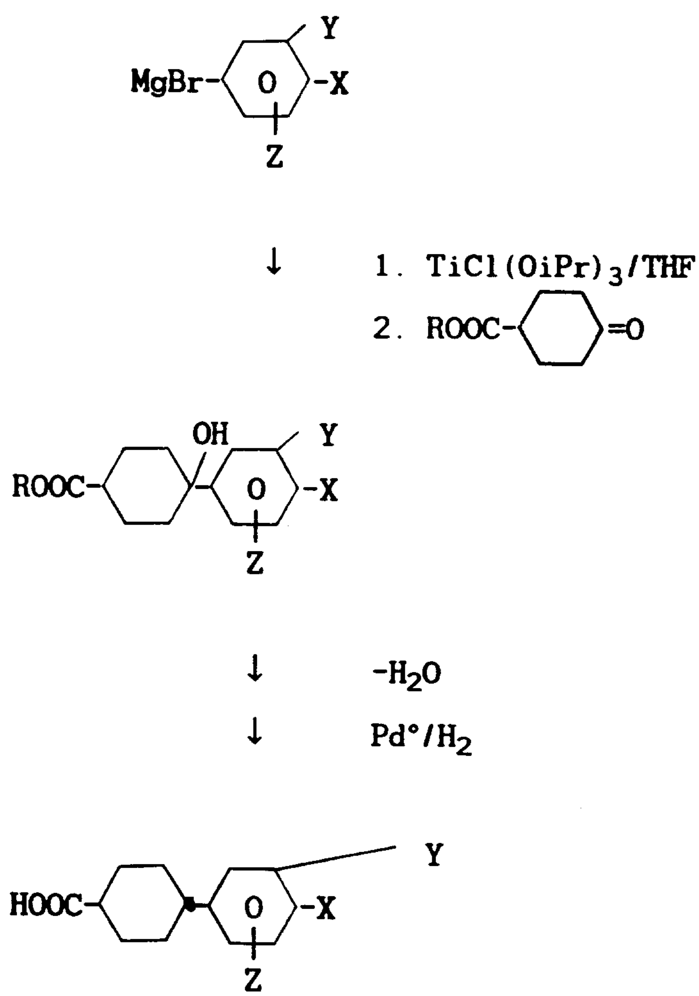

Schema 2:

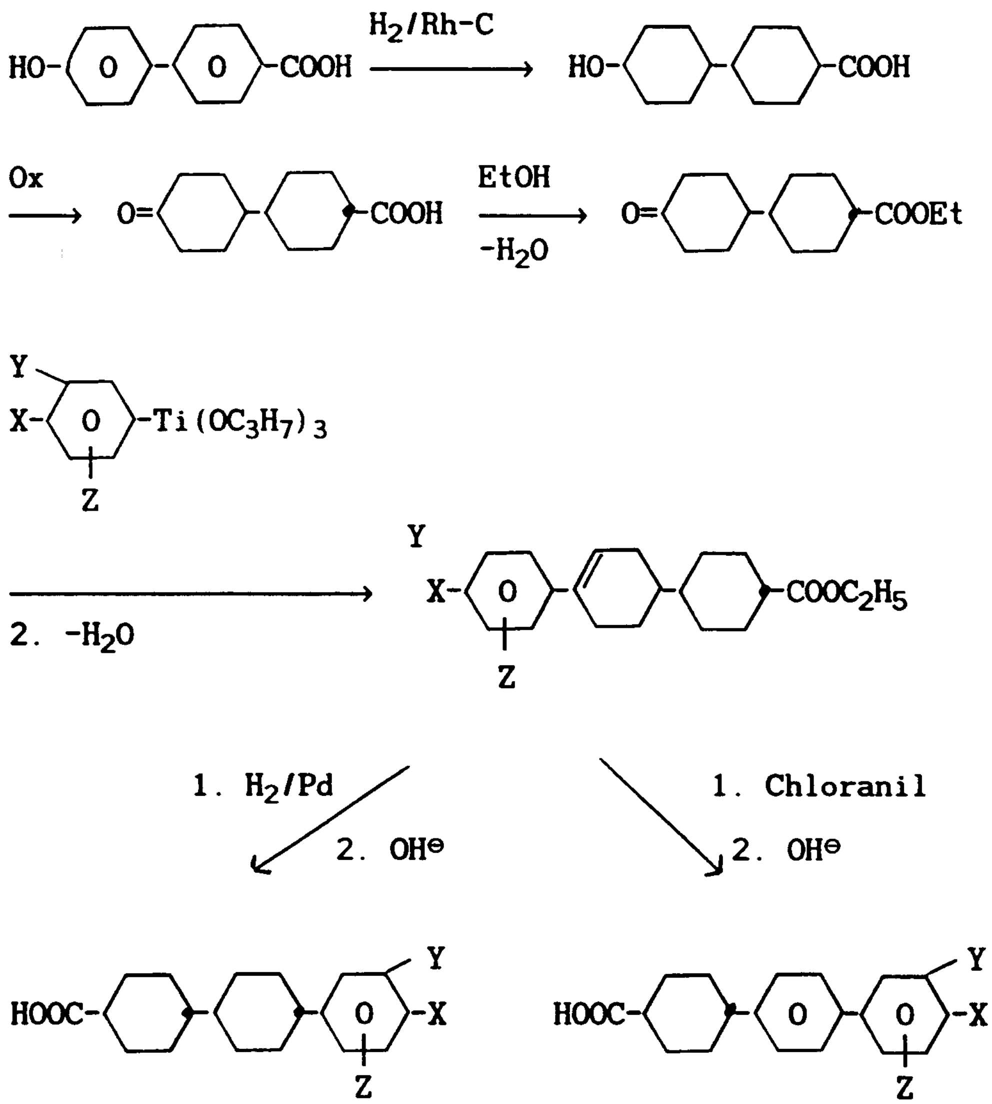


Die aufgeführten Carbonsäuren können durch übliche Homologisierungsreaktionen in die höheren Homologen (r = 1-5) übergeführt werden.

Im Falle $Q^1$ = $CH_3$ ist n vorzugsweise 1. Zur Synthese dieser bevorzugten Verbindungen wird vorzugsweise ein Phosphoniumsalz der Formel II" mit Aceton nach Wittig in Gegenwart einer Base (z.B. BuLi) zu einer erfindungsgemäßen Verbindung umgesetzt.

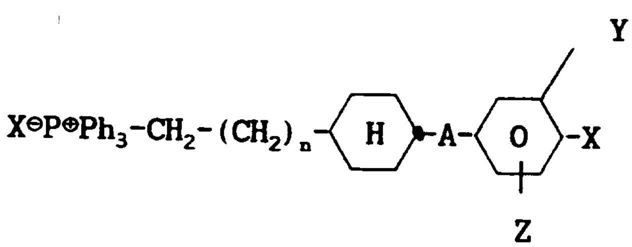

II"

Die Ausgangsstoffe der Formel II" sind zum Teil bekannt, zum Teil können sie ohne Schwierigkeiten nach Standardverfahren der organischen Chemie aus literaturbekannten Verbindungen hergestellt werden. Beispielsweise sind Vorstufen der Verbindungen der Formel II" nach folgenden Syntheseschemata erhältlich:

Schema 3:

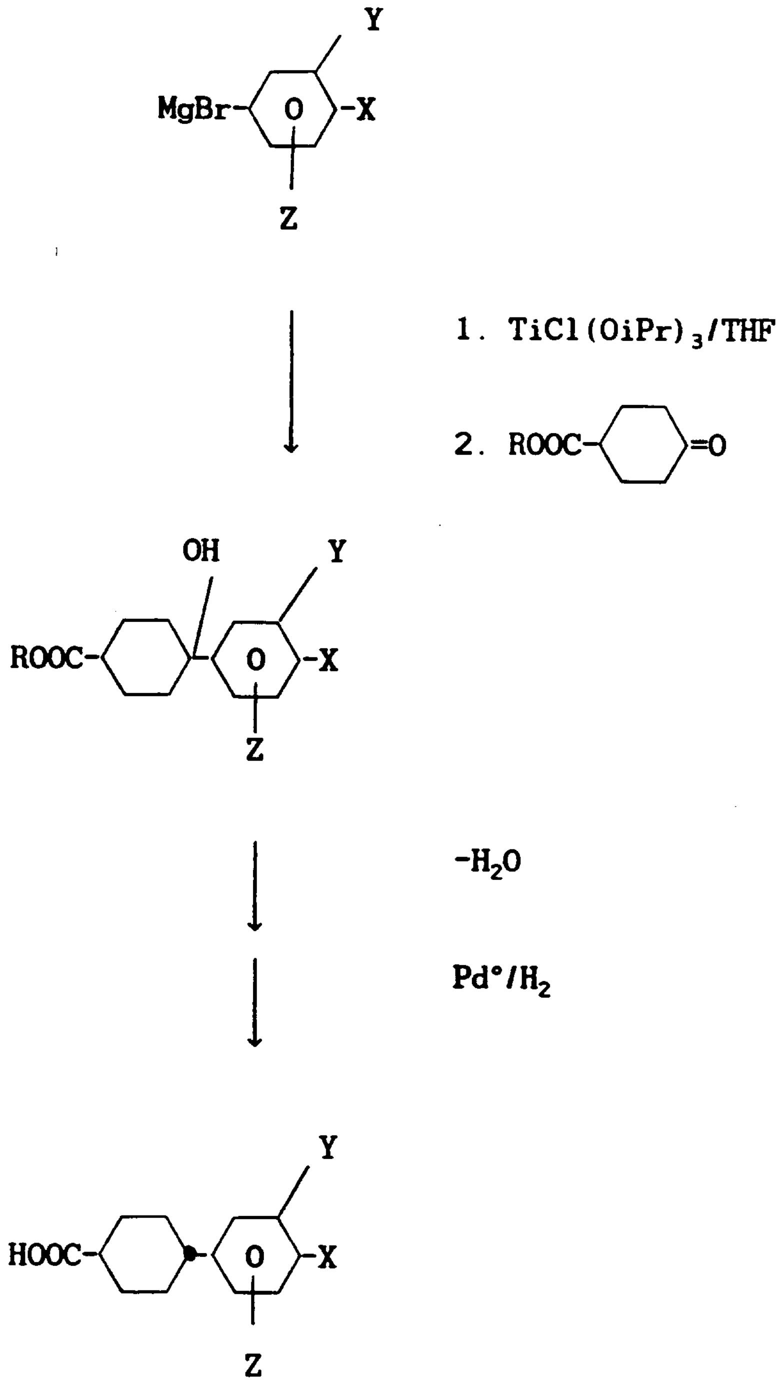

Schema 4:

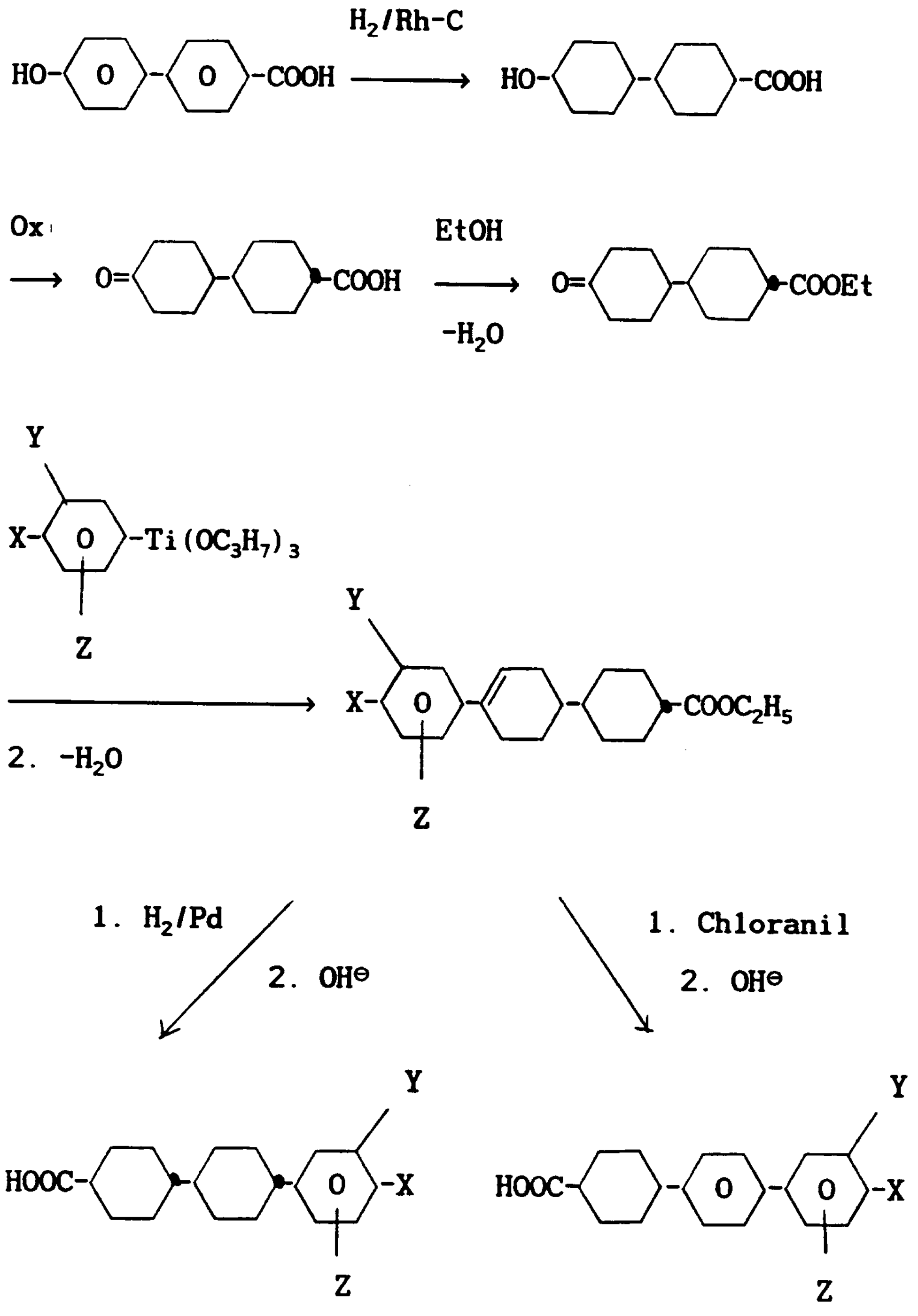


Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R''    1

R'-L-COO-E-R''    2

R'-L-OOC-E-R''    3

R'-L-$CH_2CH_2$-E-R''    4

R'-L-CC-E-R''    5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc-sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b be~ deutet R'' -CN, -$CF_3$, -$OCF_3$, -$OCHF_2$, F, Cl oder -NCS; R hat dabei die bei den Verbindungen der Teilformeln 1a bis 5a angegebene Bedeutung und ist vorzugsweise Alkyl oder Alkenyl. Besonders bevorzugt ist R'', ausgewählt aus der Gruppe bestehend aus -F, Cl, -$CF_3$, -$OCHF_2$ und -$OCF_3$. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %, Gruppe 2: 10 bis 80 %, insbesondere 10 bis 50 %, wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher

bekannt gewordenen Arten von Flüssigkristallanzeigeelementen verwendet werden können.

Derartige Zusätze sind des Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Insbesondere eignen sich die erfindungsgemäßen Medien zur Verwendung in MFK-Anzeigen.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTSOH | p-Toluolsulfonsäure |

Beispiel 1

Ein Gemisch von trans-4-(3,4-Difluorphenyl)-cyclohexancarbaldehyd und Propyltriphenylphosphoniumbromid in n-Butanol wird mit einem Äquivalent Kalium-t.butylat versetzt und wie üblich aufgearbeitet. Man erhält 4-trans-4-(1-Butenyl)-cyclohexyl-1,2-difluorbenzol.

Beispiel 2

Ein Gemisch von 2-trans-4-(p-Trifluormethozyphenyl)-cyclohexyl-ethyltriphenylphosphoniumjodid und Propanal in Hexan wird mit Butyllithium behandelt und wie üblich aufgearbeitet. Das erhaltene 4-trans-4-(cis-2-pentenyl)-cyclohexyltrifluormethoxybenzol wird mit m-Chlorperbenzoesäure epoxidiert. Zu einer Lösung des erhaltenen Epoxids (0,005 mol) in 15 ml THF gibt man unter peinlichstem Ausschluß von Luftsauerstoff 4,15 ml einer 1,15 M Lösung von Lithium-Diphenylphosphid in THF bei 25 °C.

Man wartet bis die rote Farbe des Phosphids verschwunden ist und gibt dann zu der Reaktionsmischung 1,5 Äquivalente frisch destilliertes Methyljodid (ebenfalls bei 25 °C). Nach 2 Stunden arbeitet man wäßrig auf. Die organische Phase wird abgetrennt, über Kieselgel filtriert und anschließend eingeengt. Der Rückstand wird durch Chromatographie und Kristallisation gereinigt. Man erhält 4-trans-4-(trans-2-pentenyl)-cyclohexyl-trifluormethoxybenzol.

Beispiel 3 bis 146

Analog Beispiel 1 bzw. 2 erhält man aus den entsprechenden Aldehyden die folgenden Verbindungen der Formel I:

| | n | r | X | Y | Z | A |
|---|---|---|---|---|---|---|
| ( 3) | 3 | 0 | F | F | H | '–' |
| ( 4) | 2 | 1 | F | F | H | '–' |
| ( 5) | 0 | 2 | F | F | H | '–' |
| ( 6) | 1 | 2 | F | F | H | '–' |
| ( 7) | 3 | 2 | F | F | H | '–' |

| | | | | | | |
|---|---|---|---|---|---|---|
| ( 8) | 0 | 0 | F | H | H | '-' |
| ( 9) | 1 | 0 | F | H | H | '-' |
| ( 10) | 0 | 2 | F | H | H | '-' |
| ( 11) | 1 | 2 | F | H | H | '-' |
| ( 12) | 3 | 2 | F | H | H | '-' |
| ( 13) | 0 | 0 | Cl | H | H | '-' |
| ( 14) | 1 | 0 | Cl | H | H | '-' |
| ( 15) | 0 | 2 | Cl | H | H | '-' |
| ( 16) | 1 | 2 | Cl | H | H | '-' |
| ( 17) | 3 | 2 | Cl | H | H | '-' |
| ( 18) | 0 | 0 | Cl | F | H | '-' |
| ( 19) | 1 | 0 | Cl | F | H | '-' |
| ( 20) | 0 | 2 | Cl | F | H | '-' |
| ( 21) | 1 | 2 | Cl | F | H | '-' |
| ( 22) | 3 | 2 | Cl | H | H | '-' |
| ( 23) | 0 | 0 | $-CF_3$ | H | H | '-' |
| ( 24) | 1 | 0 | $-CF_3$ | H | H | '-' |
| ( 25) | 0 | 2 | $-CF_3$ | H | H | '-' |
| ( 26) | 1 | 2 | $-CF_3$ | H | H | '-' |
| ( 27) | 3 | 2 | $-CF_3$ | H | H | '-' |
| ( 28) | 0 | 0 | $-OCF_3$ | H | H | '-' |
| ( 29) | 1 | 0 | $-OCF_3$ | H | H | '-' |
| ( 30) | 0 | 2 | $-OCF_3$ | H | H | '-' |
| ( 31) | 1 | 2 | $-OCF_3$ | H | H | '-' |
| ( 32) | 3 | 2 | $-OCF_3$ | H | H | '-' |
| ( 33) | 0 | 0 | $-OCHF_3$ | H | H | '-' |
| ( 34) | 1 | 2 | $-OCHF_3$ | H | H | '-' |

| | | | | | | |
|---|---|---|---|---|---|---|
| ( 35) | 0 | 2 | $-OCHF_3$ | H | H | '-' |
| ( 36) | 1 | 2 | $-OCHF_3$ | H | H | '-' |
| ( 37) | 3 | 2 | $-OCHF_3$ | H | H | '-' |
| ( 38) | 0 | 0 | -CN | F | H | '-' |
| ( 39) | 1 | 0 | -CN | F | H | '-' |
| ( 40) | 0 | 2 | | F | H | '-' |
| ( 41) | 1 | 2 | -CN | F | H | '-' |
| ( 42) | 3 | 2 | -CN | F | H | '-' |
| ( 43) | 0 | 0 | -CN | F | F* | '-' |
| ( 44) | 1 | 0 | -CN | F | F* | '-' |
| ( 45) | 0 | 2 | -CN | F | F* | '-' |
| ( 46) | 1 | 2 | -CN | F | F* | '-' |
| ( 47) | 3 | 2 | -CN | F | F* | '-' |

* Z in ortho-Position zu X

| | n | r | X | Y | Z | A |
|---|---|---|---|---|---|---|
| ( 57) | 3 | 0 | F | F | H | Cyc |
| ( 58) | 2 | 1 | F | F | H | Cyc |
| ( 59) | 0 | 2 | F | F | H | Cyc |
| ( 60) | 1 | 2 | F | F | H | Cyc |
| ( 61) | 3 | 2 | F | F | H | Cyc |
| ( 62) | 0 | 0 | F | H | H | Cyc |
| ( 63) | 1 | 0 | F | H | H | Cyc |
| ( 64) | 0 | 2 | F | H | H | Cyc |
| ( 65) | 1 | 2 | F | H | H | Cyc |

| | | | | | | |
|---|---|---|---|---|---|---|
| ( 66) | 3 | 2 | F | H | H | Cyc |
| ( 67) | 0 | 0 | Cl | H | H | Cyc |
| ( 68) | 1 | 0 | Cl | H | H | Cyc |
| ( 69) | 0 | 2 | Cl | H | H | Cyc |
| ( 70) | 1 | 2 | Cl | H | H | Cyc |
| ( 71) | 3 | 2 | Cl | H | H | Cyc |
| ( 72) | 0 | 0 | Cl | F | H | Cyc |
| ( 73) | 1 | 0 | Cl | F | H | Cyc |
| ( 74) | 0 | 2 | Cl | F | H | Cyc |
| ( 75) | 1 | 2 | Cl | F | H | Cyc |
| ( 76) | 3 | 2 | Cl | F | H | Cyc |
| ( 77) | 0 | 0 | $-CF_3$ | H | H | Cyc |
| ( 78) | 1 | 0 | $-CF_3$ | H | H | Cyc |
| ( 7) | 0 | 2 | $-CF_3$ | H | H | Cyc |
| ( 80) | 1 | 2 | $-CF_3$ | H | H | Cyc |
| ( 81) | 3 | 2 | $-CF_3$ | H | H | Cyc |
| ( 82) | 0 | 0 | $-OCF_3$ | H | H | Cyc |
| ( 83) | 1 | 0 | $-OCF_3$ | H | H | Cyc |
| ( 84) | 0 | 2 | $-OCF_3$ | H | H | Cyc |
| ( 85) | 1 | 2 | $-OCF_3$ | H | H | Cyc |
| ( 86) | 3 | 2 | $-OCF_3$ | H | H | Cyc |
| ( 87) | 0 | 0 | $-OCHF_2$ | H | H | Cyc |
| ( 88) | 1 | 0 | $-OCHF_2$ | H | H | Cyc |
| ( 89) | 0 | 2 | $-OCHF_2$ | H | H | Cyc |
| ( 90) | 1 | 2 | $-OCHF_2$ | H | H | Cyc |
| ( 91) | 3 | 2 | $-OCHF_2$ | H | H | Cyc |

| | | | | | | |
|---|---|---|---|---|---|---|
| ( 92) | 0 | 0 | -CN | F | H | Cyc |
| ( 93) | 1 | 0 | -CN | F | H | Cyc |
| ( 94) | 0 | 2 | -CN | F | H | Cyc |
| ( 95) | 1 | 2 | -CN | F | H | Cyc |
| ( 96) | 3 | 2 | -CN | F | H | Cyc |
| ( 97) | 0 | 0 | -CN | F | F* | Cyc |
| ( 98) | 1 | 0 | -CN | F | F* | Cyc |
| ( 99) | 0 | 2 | -CN | F | F* | Cyc |
| (100) | 1 | 2 | -CN | F | F* | Cyc |
| (101) | 3 | 2 | -CN | F | F* | Cyc |

* Z in ortho-Position zu X

| | n | r | X | Y | Z | A |
|---|---|---|---|---|---|---|
| (102) | 3 | 0 | F | F | H | Phe |
| (103) | 2 | 1 | F | F | H | Phe |
| (104) | 0 | 2 | F | F | H | Phe |
| (105) | 1 | 2 | F | F | H | Phe |
| (106) | 3 | 2 | F | F | H | Phe |
| (107) | 0 | 0 | F | H | H | Phe |
| (108) | 1 | 0 | F | H | H | Phe |
| (109) | 0 | 2 | F | H | H | Phe |
| (110) | 1 | 2 | F | H | H | Phe |
| (111) | 3 | 2 | F | H | H | Phe |
| (112) | 0 | 0 | Cl | H | H | Phe |
| (113) | 1 | 0 | Cl | H | H | Phe |
| (114) | 0 | 2 | Cl | H | H | Phe |
| (115) | 1 | 2 | Cl | H | H | Phe |

| | | | | | | |
|---|---|---|---|---|---|---|
| (116) | 3 | 2 | Cl | H | H | Phe |
| (117) | 0 | 0 | Cl | F | H | Phe |
| (118) | 1 | 0 | Cl | F | H | Phe |
| (119) | 0 | 2 | Cl | F | H | Phe |
| (120) | 1 | 2 | Cl | F | H | Phe |
| (121) | 3 | 2 | Cl | F | H | Phe |
| (122) | 0 | 0 | $-CF_3$ | H | H | Phe |
| (123) | 1 | 0 | $-CF_3$ | H | H | Phe |
| (124) | 0 | 2 | $-CF_3$ | H | H | Phe |
| (125) | 1 | 2 | $-CF_3$ | H | H | Phe |
| (126) | 3 | 2 | $-CF_3$ | H | H | Phe |
| (127) | 0 | 0 | $-OCF_3$ | H | H | Phe |
| (128) | 1 | 0 | $-OCF_3$ | H | H | Phe |
| (129) | 0 | 2 | $-OCF_3$ | H | H | Phe |
| (130) | 1 | 2 | $-OCF_3$ | H | H | Phe |
| (131) | 3 | 2 | $-OCF_3$ | H | H | Phe |
| (132) | 0 | 0 | $-OCHF_2$ | H | H | Phe |
| (133) | 1 | 0 | $-OCHF_2$ | H | H | Phe |
| (134) | 0 | 2 | $-OCHF_2$ | H | H | Phe |
| (135) | 1 | 2 | $-OCHF_2$ | H | H | Phe |
| (136) | 3 | 2 | $-OCHF_2$ | H | H | Phe |
| (137) | 0 | 0 | -CN | F | H | Phe |
| (138) | 1 | 0 | -CN | F | H | Phe |
| (139) | 0 | 2 | -CN | F | H | Phe |
| (140) | 1 | 2 | -CN | F | H | Phe |

| | | | | | | |
|---|---|---|---|---|---|---|
| (141) | 3 | 2 | -CN | F | H | Phe |
| (142) | 0 | 0 | -CN | F | F[x] | Phe |
| (143) | 1 | 0 | -CN | F | F[x] | Phe |
| (144) | 0 | 2 | -CN | F | F[x] | Phe |
| (145) | 1 | 2 | -CN | F | F[x] | Phe |
| (146) | 3 | 2 | -CN | F | F[x] | Phe |

[x] Z in ortho-Position zu X

| | r | X | Y | Z | A |
|---|---|---|---|---|---|
| (148) | 0 | F | H | H | '-' |
| (149) | 1 | F | H | H | '-' |
| (150) | 2 | F | H | H | '-' |
| (152) | 0 | F | F | H | '-' |
| (153) | 1 | F | F | H | '-' |
| (154) | 2 | F | F | H | '-' |
| (155) | 3 | F | F | H | '-' |
| (158) | 0 | Cl | H | H | '-' |
| (159) | 1 | Cl | H | H | '-' |
| (160) | 2 | Cl | H | H | '-' |
| (161) | 3 | Cl | H | H | '-' |
| (164) | 0 | Cl | F | H | '-' |
| (165) | 1 | Cl | F | H | '-' |
| (166) | 2 | Cl | F | H | '-' |
| (167) | 3 | Cl | F | H | '-' |
| (170) | 0 | $CF_3$ | H | H | '-' |
| (171) | 1 | $CF_3$ | H | H | '-' |
| (172) | 2 | $CF_3$ | H | H | '-' |
| (173) | 3 | $CF_3$ | H | H | '-' |

| | | | | | |
|---|---|---|---|---|---|
| (176) | 0 | $OCF_3$ | H | H | '-' |
| (177) | 1 | $OCF_3$ | H | H | '-' |
| (178) | 2 | $OCF_3$ | H | H | '-' |
| (179) | 0 | $OCHF_2$ | H | H | '-' |
| (180) | 1 | $OCHF_2$ | H | H | '-' |
| (181) | 2 | $OCHF_2$ | H | H | '-' |
| (182) | 0 | F | H | H | Cyc |
| (183) | 1 | F | H | H | Cyc |
| (184) | 2 | F | H | H | Cyc |
| (186) | 0 | F | F | H | Cyc |
| (187) | 1 | F | F | H | Cyc |
| (188) | 2 | F | F | H | Cyc |
| (192) | 0 | Cl | H | H | Cyc |
| (193) | 1 | Cl | H | H | Cyc |
| (194) | 2 | Cl | H | H | Cyc |
| (198) | 0 | Cl | F | H | Cyc |
| (199) | 1 | Cl | F | H | Cyc |
| (200) | 2 | Cl | F | H | Cyc |

| | | | | | |
|---|---|---|---|---|---|
| (204) | 0 | $CF_3$ | H | H | Cyc |
| (205) | 1 | $CF_3$ | H | H | Cyc |
| (206) | 2 | $CF_3$ | H | H | Cyc |
| (210) | 1 | $OCF_3$ | H | H | Cyc |
| (211) | 2 | $OCF_3$ | H | H | Cyc |
| (213) | 0 | $OCHF_2$ | H | H | Cyc |
| (214) | 1 | $OCHF_2$ | H | H | Cyc |
| (215) | 2 | $OCHF_2$ | H | H | Cyc |
| (216) | 0 | F | H | H | Phe |
| (217) | 1 | F | H | H | Phe |
| (218) | 2 | F | H | H | Phe |
| (219) | 3 | F | H | H | Phe |
| (220) | 0 | F | F | H | Phe |
| (221) | 1 | F | F | H | Phe |
| (222) | 2 | F | F | H | Phe |
| (223) | 3 | F | F | H | Phe |
| (226) | 0 | Cl | H | H | Phe |
| (227) | 1 | Cl | H | H | Phe |
| (228) | 2 | Cl | H | H | Phe |
| (229) | 3 | Cl | H | H | Phe |

| | | | | | |
|---|---|---|---|---|---|
| (232) | 0 | Cl | F | H | Phe |
| (233) | 1 | Cl | F | H | Phe |
| (234) | 2 | Cl | F | H | Phe |
| (235) | 3 | Cl | F | H | Phe |
| (238) | 0 | $CF_3$ | H | H | Phe |
| (239) | 1 | $CF_3$ | H | H | Phe |
| (240) | 2 | $CF_3$ | H | H | Phe |
| (241) | 3 | $CF_3$ | H | H | Phe |
| (244) | 1 | $OCF_3$ | H | H | Phe |
| (245) | 2 | $OCF_3$ | H | H | Phe |
| (246) | 3 | $OCF_3$ | H | H | Phe |
| (247) | 1 | $OCHF_2$ | H | H | Phe |
| (248) | 2 | $OCHF_2$ | H | H | Phe |
| (249) | 3 | $OCHF_2$ | H | H | Phe |

Beispiel 250:

Durch Umsetzung von 2-trans-4-(p-Fluorphenyl)-cyclohexyl]-ethylphosphoniumiodid mit Aceton und Butyllithium nach Wittig erhält man nach üblicher Aufarbeitung und Aufreinigung durch Chromatographie trans-1-p-Fluorphenyl-4-(4-methylpenten-3-yl)-cyclohexan.

Beispiele 251 - 352:

Analog Beispiel 250 erhält man aus den entsprechenden Aldehyden die folgenden Verbindungen (n = 1, $Q^1$ = $CH_3$, $Q^2$ = H):

| | r | X | Y | Z | A |
|---|---|---|---|---|---|
| (251) | 0 | F | H | H | '-' |
| (252) | 1 | F | H | H | '-' |
| (253) | 2 | F | H | H | '-' |
| (254) | 4 | F | H | H | '-' |
| (255) | 0 | F | F | H | '-' |
| (256) | 1 | F | F | H | '-' |
| (257) | 2 | F | F | H | '-' |
| (258) | 3 | F | F | H | '-' |
| (261) | 0 | Cl | H | H | '-' |
| (262) | 1 | Cl | H | H | '-' |
| (263) | 2 | Cl | H | H | '-' |
| (264) | 3 | Cl | H | H | '-' |

| | r | X | Y | Z | A |
|---|---|---|---|---|---|
| (267) | 0 | Cl | F | H | '-' |
| (268) | 1 | Cl | F | H | '-' |
| (269) | 2 | Cl | F | H | '-' |
| (270) | 3 | Cl | F | H | '-' |
| (273) | 0 | $CF_3$ | H | H | '-' |
| (274) | 1 | $CF_3$ | H | H | '-' |
| (275) | 2 | $CF_3$ | H | H | '-' |
| (276) | 3 | $CF_3$ | H | H | '-' |
| (279) | 0 | $OCF_3$ | H | H | '-' |
| (280) | 1 | $OCF_3$ | H | H | '-' |
| (281) | 2 | $OCF_3$ | H | H | '-' |
| (282) | 0 | $OCHF_2$ | H | H | '-' |
| (283) | 1 | $OCHF_2$ | H | H | '-' |
| (284) | 2 | $OCHF_2$ | H | H | '-' |
| (285) | 0 | F | H | H | Cyc |
| (286) | 1 | F | H | H | Cyc |
| (287) | 2 | F | H | H | Cyc |
| (289) | 0 | F | F | H | Cyc |

| | r | X | Y | Z | A |
|---|---|---|---|---|---|
| (290) | 1 | F | F | H | Cyc |
| (291) | 2 | F | F | H | Cyc |
| (295) | 0 | Cl | H | H | Cyc |
| (296) | 1 | Cl | H | H | Cyc |
| (297) | 2 | Cl | H | H | Cyc |
| (301) | 0 | Cl | F | H | Cyc |
| (302) | 1 | Cl | F | H | Cyc |
| (303) | 2 | Cl | F | H | Cyc |
| (307) | 0 | $CF_3$ | H | H | Cyc |
| (308) | 1 | $CF_3$ | H | H | Cyc |
| (309) | 2 | $CF_3$ | H | H | Cyc |
| (313) | 1 | $OCF_3$ | H | H | Cyc |
| (314) | 2 | $OCF_3$ | H | H | Cyc |

| | r | X | Y | Z | A |
|---|---|---|---|---|---|
| (316) | 0 | $OCHF_2$ | H | H | Cyc |
| (317) | 1 | $OCHF_2$ | H | H | Cyc |
| (318) | 2 | $OCHF_2$ | H | H | Cyc |
| (319) | 0 | F | H | H | Phe |
| (320) | 1 | F | H | H | Phe |
| (321) | 2 | F | H | H | Phe |
| (322) | 3 | F | H | H | Phe |
| (323) | 0 | F | F | H | Phe |
| (324) | 1 | F | F | H | Phe |
| (325) | 2 | F | F | H | Phe |
| (326) | 3 | F | F | H | Phe |
| (329) | 0 | Cl | H | H | Phe |
| (330) | 1 | Cl | H | H | Phe |
| (331) | 2 | Cl | H | H | Phe |
| (332) | 3 | Cl | H | H | Phe |
| (335) | 0 | Cl | F | H | Phe |
| (336) | 1 | Cl | F | H | Phe |
| (337) | 2 | Cl | F | H | Phe |
| (338) | 3 | Cl | F | H | Phe |
| (341) | 0 | $CF_3$ | H | H | Phe |

| | r | X | Y | Z | A |
|---|---|---|---|---|---|
| (342) | 1 | $CF_3$ | H | H | Phe |
| (343) | 2 | $CF_3$ | H | H· | Phe |
| (344) | 3 | $CF_3$ | H | H | Phe |
| (347) | 1 | $OCF_3$ | H | H | Phe |
| (348) | 2 | $OCF_3$ | H | H | Phe |
| (349) | 3 | $OCF_3$ | H | H | Phe |
| (350) | 1 | $OCHF_2$ | H | H | Phe |
| (351) | 2 | $OCHF_2$ | H | H | Phe |
| (352) | 3 | $OCHF_2$ | H | H | Phe |

Es folgen Beispiele für Medien mit einem Gehalt an mindestens einer Verbindung der Formel I:

Beispiel A

Ein Gemisch aus
7 % p-(trans-4-Propylcyclohexyl)-benzonitril,
5 % 4-[trans-4-(trans-2-pentenyl)-cyclohexyl]-trifluormethoxybenzol,
24 % p-(trans-4-Pentylcyclohexyl)-fluorbenzol,
14 % p-(trans-4-Heptylcyclohexyl)-fluorbenzol,
15 % 2-[trans-4-(3,4-Difluorphenyl)-cyclohexyl]-5-butyl-1,3-dioxan,
18 % 2-[trans-4-(p-fluorphenyl)-cyclohexyl]-5-ethyl-1,3-dioxan und
17 % 2-[trans-4-(p-fluorphenyl)-cyclohexyl]-5-propyl-1,3-dioxan
zeigt einen hohen elektrischen Widerstand.

**Patentansprüche**

1. Flüssigkristallines Medium für elektrooptische Anzeigen mit mindestens zwei flüssigkristallinen Komponenten, dadurch gekennzeichnet, daß mindestens eine Komponente ein Phenylcyclohexan der Formel I 1 ist,

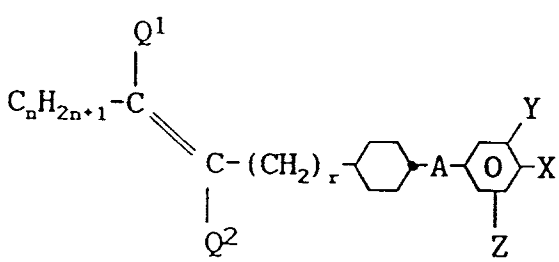

worin n 0 bis 7, $Q^1$ und $Q^2$ H, r 0, 1 oder 2, A trans-1,4-Cyclohexylen, 1,4-Phenylen, 3-Fluor-1,4-phenylen oder eine Einfachbindung, X F, Cl, $-CF_3$, -CN, $-OCF_3$ oder $-OCHF_2$, und Y und Z jeweils unabhängig voneinander H oder F bedeuten, mit der Maßgabe, daß im Falle X = CN Y und/oder Z F bedeuten, und es als weiteren Bestandteil eine Verbindung der Formel 5 enthält,

R'-L-C≡C-E-R"  5

worin L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc-sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, und

R' und R" jeweils unabhängig voneinander Alkyl, Alkenyl, Alkoxy, Alkenyloxy oder Alkanoyloxy mit bis zu 8 Kohlenstoffatomen,

oder R" auch -CN, $-CF_3$, $-OCF_3$, $-OCHF_2$, F, Cl oder -NCS ist.

2. Phenylcyclohexane nach Anspruch 1, dadurch gekennzeichnet, daß Y = F, Z = H oder F und r = 0, bedeutet.

3. Phenylcyclohexane nach Anspruch 2, dadurch gekennzeichnet, daß X F, Cl, $-CF_3$, $-OCHF_2$ oder $-OCF_3$ bedeutet.

4. Phenylcyclohexane nach Anspruch 1, dadurch gekennzeichnet, daß r 0 und X = Cl, $-OCF_3$ oder $-OCHF_2$ bedeutet.

5. Phenylcyclohexane nach Anspruch 1, dadurch gekennzeichnet, daß

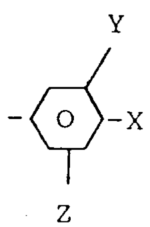

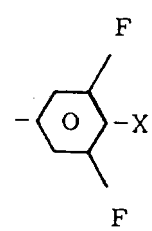

bedeutet.

6. Phenylcyclohexane nach einem der Ansprüche 1, 2, 3, 4 oder 5, dadurch gekennzeichnet, daß X - $CF_3$, $-OCF_3$ oder - $OCHF_2$ bedeutet.

7. Phenylcyclohexane nach Anspruch 5, dadurch gekennzeichnet, daß X = CN, Z = F und Y = H oder F.

8. Phenylcyclohexane der Formel I

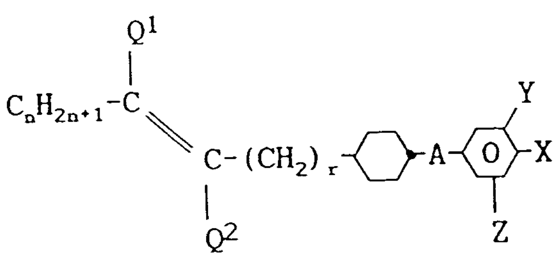

worin n 0 bis 7, $Q^1$ und $Q^2$ H, r 2 oder 3, A 1,4-Phenylen, 3-Fluor-1,4-phenylen oder eine Einfachbindung, X F oder Cl, Z = H und Y = F oder H bedeutet.

**9.** 4-trans-4-(1-Butenyl)-cyclohexyl-1,2-difluorbenzol.

**10.** Phenylcyclohexane nach Anspruch 1, dadurch gekennzeichnet, daß n, r, X, Y, Z und A folgende Bedeutungen haben:

| | n | r | X | Y | Z | A |
|---|---|---|---|---|---|---|
| (3) | 3 | 0 | F | F | H | '-' |
| (8) | 0 | 0 | F | H | H | '-' |
| (9) | 1 | 0 | F | H | H | '-' |
| (13) | 0 | 0 | Cl | H | H | '-' |
| (14) | 1 | 0 | Cl | H | H | '-' |
| (18) | 0 | 0 | Cl | F | H | '-' |
| (19) | 1 | 0 | Cl | F | H | '-' |
| (23) | 0 | 0 | $-CF_3$ | H | H | '-' |
| (24) | 1 | 0 | $-CF_3$ | H | H | '-' |
| (25) | 0 | 2 | $-CF_3$ | H | H | '-' |
| (26) | 1 | 2 | $-CF_3$ | H | H | '-' |
| (27) | 3 | 2 | $-CF_3$ | H | H | '-' |
| (28) | 0 | 0 | $-OCF_3$ | H | H | '-' |
| (29) | 1 | 0 | $-OCF_3$ | H | H | '-' |
| (30) | 0 | 2 | $-OCF_3$ | H | H | '-' |
| (31) | 1 | 2 | $-OCF_3$ | H | H | '-' |
| (32) | 3 | 2 | $-OCF_3$ | H | H | '-' |
| (33) | 0 | 0 | $-OCHF_3$ | H | H | '-' |
| (34) | 1 | 2 | $-OCHF_3$ | H | H | '-' |
| (35) | 0 | 2 | $-OCHF_3$ | H | H | '-' |
| (36) | 1 | 2 | $-OCHF_3$ | H | H | '-' |
| (37) | 3 | 2 | $-OCHF_3$ | H | H | '-' |
| (43) | 0 | 0 | -CN | F | $F^x$ | '-' |
| (44) | 1 | 0 | -CN | F | $F^x$ | '-' |
| (45) | 0 | 2 | -CN | F | $F^x$ | '-' |
| (46) | 1 | 2 | -CN | F | $F^x$ | '-' |
| (47) | 3 | 2 | -CN | F | $F^x$ | '-' |

$^x$ Z in ortho-Position zu X

**11.** Phenylcyclohexane nach Anspruch 1, dadurch gekennzeichnet, daß n, r, X, Y, Z und A folgende Bedeutungen haben:

| | n | r | X | Y | Z | A |
|---|---|---|---|---|---|---|
| (57) | 3 | 0 | F | F | H | Cyc |
| (67) | 0 | 0 | Cl | H | H | Cyc |
| (68) | 1 | 0 | Cl | H | H | Cyc |
| (72) | 0 | 0 | Cl | F | H | Cyc |
| (73) | 1 | 0 | Cl | F | H | Cyc |
| (77) | 0 | 0 | $-CF_3$ | H | H | Cyc |
| (78) | 1 | 0 | $-CF_3$ | H | H | Cyc |
| (79) | 0 | 2 | $-CF_3$ | H | H | Cyc |
| (80) | 1 | 2 | $-CF_3$ | H | H | Cyc |
| (81) | 3 | 2 | $-CF_3$ | H | H | Cyc |
| (82) | 0 | 0 | $-OCF_3$ | H | H | Cyc |
| (83) | 1 | 0 | $-OCF_3$ | H | H | Cyc |
| (84) | 0 | 2 | $-OCF_3$ | H | H | Cyc |
| (85) | 1 | 2 | $-OCF_3$ | H | H | Cyc |
| (86) | 3 | 2 | $-OCF_3$ | H | H | Cyc |
| (87) | 0 | 0 | $-OCHF_2$ | H | H | Cyc |
| (88) | 1 | 0 | $-OCHF_2$ | H | H | Cyc |
| (89) | 0 | 2 | $-OCHF_2$ | H | H | Cyc |
| (90) | 1 | 2 | $-OCHF_2$ | H | H | Cyc |
| (91) | 3 | 2 | $-OCHF_2$ | H | H | Cyc |
| (97) | 0 | 0 | -CN | F | $F^x$ | Cyc |
| (98) | 1 | 0 | -CN | F | $F^x$ | Cyc |
| (99) | 0 | 2 | -CN | F | $F^x$ | Cyc |
| (100) | 1 | 2 | -CN | F | $F^x$ | Cyc |
| (101) | 3 | 2 | -CN | F | $F^x$ | Cyc |

$^x$ Z in ortho-Position zu X

**12.** Phenylcyclohexane nach Anspruch 1, dadurch gekennzeichnet, daß n, r, X, Y, Z und A folgende Bedeutungen haben:

| | n | r | X | Y | Z | A |
|---|---|---|---|---|---|---|
| (102) | 3 | 0 | F | F | H | Phe |
| (112) | 0 | 0 | Cl | H | H | Phe |
| (113) | 1 | 0 | Cl | H | H | Phe |
| (117) | 0 | 0 | Cl | F | H | Phe |
| (118) | 1 | 0 | Cl | F | H | Phe |
| (122) | 0 | 0 | $-CF_3$ | H | H | Phe |
| (123) | 1 | 0 | $-CF_3$ | H | H | Phe |
| (124) | 0 | 2 | $-CF_3$ | H | H | Phe |
| (125) | 1 | 2 | $-CF_3$ | H | H | Phe |
| (126) | 3 | 2 | $-CF_3$ | H | H | Phe |
| (127) | 0 | 0 | $-OCF_3$ | H | H | Phe |
| (128) | 1 | 0 | $-OCF_3$ | H | H | Phe |
| (129) | 0 | 2 | $-OCF_3$ | H | H | Phe |
| (130) | 1 | 2 | $-OCF_3$ | H | H | Phe |
| (131) | 3 | 2 | $-OCF_3$ | H | H | Phe |
| (132) | 0 | 0 | $-OCHF_2$ | H | H | Phe |
| (133) | 1 | 0 | $-OCHF_2$ | H | H | Phe |
| (134) | 0 | 2 | $-OCHF_2$ | H | H | Phe |
| (135) | 1 | 2 | $-OCHF_2$ | H | H | Phe |
| (136) | 3 | 2 | $-OCHF_2$ | H | H | Phe |
| (137) | 0 | 0 | -CN | F | H | Phe |
| (138) | 1 | 0 | -CN | F | H | Phe |
| (139) | 0 | 2 | -CN | F | H | Phe |
| (140) | 1 | 2 | -CN | F | H | Phe |
| (141) | 3 | 2 | -CN | F | H | Phe |
| (142) | 0 | 0 | -CN | F | $F^x$ | Phe |
| (143) | 1 | 0 | -CN | F | $F^x$ | Phe |
| (144) | 0 | 2 | -CN | F | $F^x$ | Phe |
| (145) | 1 | 2 | -CN | F | $F^x$ | Phe |
| (146) | 3 | 2 | -CN | F | $F^x$ | Phe |

$^x$ Z in ortho-Position zu X

**13.** Verfahren zur Herstellung von Phenylcyclohexanen nach Anspruch 2, dadurch gekennzeichnet, daß entweder ein Aldehyd der Formel II

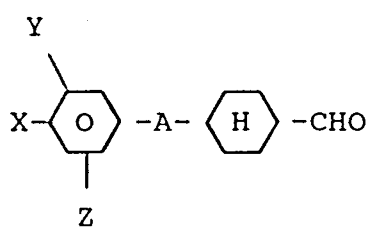

II

mit einem Phosphoniumsalz nach Wittig umgesetzt wird oder ein Phosphoniumsalz der Formel III

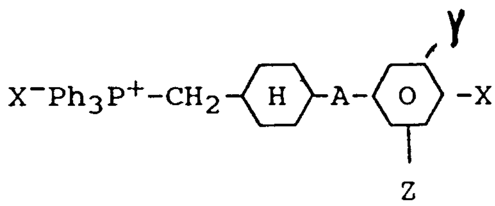 III

nach Wittig mit einem entsprechenden Aldehyd umgesetzt wird.

**14.** Verfahren zur Herstellung von Phosphoniumsalzen der Formel III nach Anspruch 13, dadurch gekennzeichnet, daß man einen Cyclohexylmethylalkohol der Formel

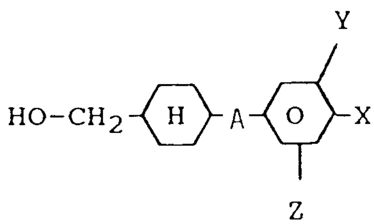

nach Überführung in das Cyclohexylmethyljodid mit Triphenylphosphin umsetzt.

**15.** Cyclohexylmethylalkohol der Formel

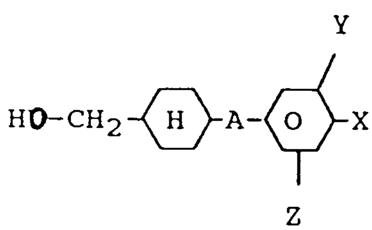

worin Y = F, Z = H oder F, X = F, Cl, $-CF_3$, -CN, $-OCF_3$ oder $OCHF_2$ und A trans-1,4-Cyclohexylen, 1,4-Phenylen, 3-Fluor-1,4-phenylen oder eine Einfachbindung ist.

**16.** Zwischenprodukt der Formel II

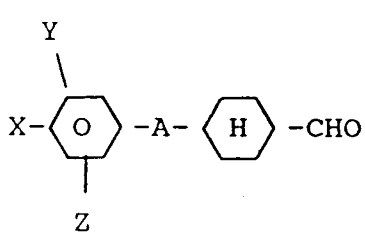

worin Y = F, Z = H oder F, X, F, Cl, $-CF_3$, -CN, $-OCF_3$ oder $-OCHF_2$ und A trans-1,4-cyclohexylen, 1,4-Phenylen, 3-Fluor-1,4-phenylen oder eine Einfachbindung ist.

**17.** Verwendung der Phenylcyclohexane der Formel I nach einem der Ansprüche 2 bis 12 Komponenten flüssigkristalliner Medien für elektrooptische Anzeigen.

**18.** Flüssigkristallines Medium für elektrooptische Anzeigen mit mindestens zwei flüssigkristallinen Komponenten, dadurch gekennzeichnet, daß mindestens eine Komponente ein Phenylcyclohexan nach einem der Ansprüche 2 bis 12.

**19.** Elektrooptische Anzeige auf der Basis einer Flüssigkristallzelle, dadurch gekennzeichnet, daß die Flüssigkristallzelle ein Medium nach Anspruch 1 enthält.

**20.** Elektrooptische Anzeige auf der Basis einer Flüssigkristallzelle, dadurch gekennzeichnet, daß die Flüssigkristallzelle ein Medium nach Anspruch 18 enthält.

## Claims

**1.** Liquid-crystalline medium for electrooptical displays having at least two liquid-crystalline components, characterized in that at least one component is a phenylcyclohexane of the formula I 1 [sic]

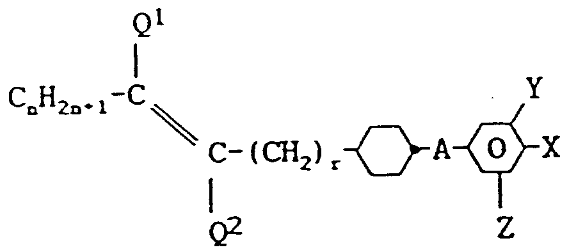

in which n is 0 to 7, $Q^1$ and $Q^2$ are H, r is 0, 1 or 2, A is trans-1,4-cyclohexylene, 1,4-phenylene, 3-fluoro-1,4-phenylene or a single bond, X is F, Cl, $-CF_3$, -CN, $-OCF_3$ or $-OCHF_2$, and Y and Z are each, independently of one another, H or F, with the proviso that, in the case where X = CN, Y and/or Z are F, and it [sic] contains as further constituent a compound of the formula 5,

R'-L-C≡C-E-R''      5

in which L and E, which may be identical or different, are each independently of one another a bivalent radical from the group consisting of -Phe-, -Cyc-, -Phe-Phe-, -Phe-Cyc-, -Cyc-Cyc-, -Pyr-, -Dio-, -G-Phe- and -G-Cyc-and their mirror images, where Phe is 1,4-phenylene which is unsubstituted or is substituted by fluoro, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl, and
R' and R'' are each independently of the other alkyl, alkenyl, alkoxy, alkenyloxy or alkanoyloxy having up to 8 carbon atoms, or alternatively R'' is -CN, $-CF_3$, $-OCF_3$, $-OCHF_2$, F, Cl or -NCS.

**2.** Phenylcyclohexanes according to Claim 1, characterized in that Y = F, Z = H or F and r = 0.

**3.** Phenylcyclohexanes according to Claim 2, characterized in that X is F, Cl, $-CF_3$, $-OCHF_2$ or $-OCF_3$.

**4.** Phenylcyclohexanes according to Claim 1, characterized in that r is 0 and X = Cl, $-OCF_3$ or $-OCHF_2$.

**5.** Phenylcyclohexanes according to Claim 1, characterized in that

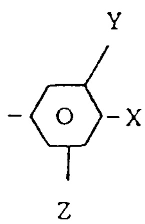

is

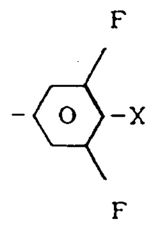

.

**6.** Phenylcyclohexanes according to one of Claims 1, 2, 3, 4 or 5, characterized in that X is $-CF_3$, $-OCF_3$ or $-OCHF_2$.

**7.** Phenylcyclohexanes according to Claim 5, characterized in that X = CN, Z = F and X = H or F.

**8.** Phenylcyclohexanes of the formula I

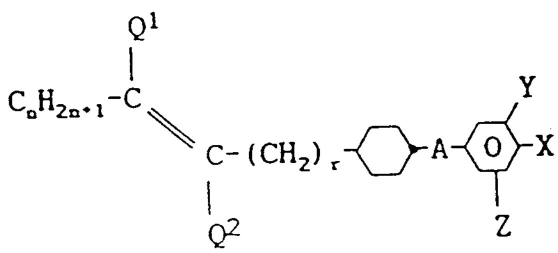

in which n is 0 to 7, $Q^1$ and $Q^2$ are H, r is 2 or 3, A is 1,4-phenylene, 3-fluoro-1,4-phenylene or a single bond, X is F or Cl, Z = H and Y = F or H.

**9.** 4-trans-4-(1-Butenyl)-cyclohexyl-1,2-difluorobenzene.

**10.** Phenylcyclohexanes according to Claim 1, characterized in that n, r, X, Y, Z and A have the following meanings:

| | n | r | X | Y | Z | A |
|---|---|---|---|---|---|---|
| (3) | 3 | 0 | F | F | H | '-' |
| (8) | 0 | 0 | F | H | H | '-' |
| (9) | 1 | 0 | F | H | H | '-' |
| (13) | 0 | 0 | Cl | H | H | '-' |
| (14) | 1 | 0 | Cl | H | H | '-' |
| (18) | 0 | 0 | Cl | F | H | '-' |
| (19) | 1 | 0 | Cl | F | H | '-' |
| (23) | 0 | 0 | $-CF_3$ | H | H | '-' |
| (24) | 1 | 0 | $-CF_3$ | H | H | '-' |
| (25) | 0 | 2 | $-CF_3$ | H | H | '-' |
| (26) | 1 | 2 | $-CF_3$ | H | H | '-' |
| (27) | 3 | 2 | $-CF_3$ | H | H | '-' |
| (28) | 0 | 0 | $-OCF_3$ | H | H | '-' |
| (29) | 1 | 0 | $-OCF_3$ | H | H | '-' |
| (30) | 0 | 2 | $-OCF_3$ | H | H | '-' |
| (31) | 1 | 2 | $-OCF_3$ | H | H | '-' |
| (32) | 3 | 2 | $-OCF_3$ | H | H | '-' |
| (33) | 0 | 0 | $-OCHF_3$ | H | H | '-' |
| (34) | 1 | 2 | $-OCHF_3$ | H | H | '-' |
| (35) | 0 | 2 | $-OCHF_3$ | H | H | '-' |
| (36) | 1 | 2 | $-OCHF_3$ | H | H | '-' |
| (37) | 3 | 2 | $-OCHF_3$ | H | H | '-' |
| (43) | 0 | 0 | -CN | F | $F^x$ | '-' |
| (44) | 1 | 0 | -CN | F | $F^x$ | '-' |
| (45) | 0 | 2 | -CN | F | $F^x$ | '-' |
| (46) | 1 | 2 | -CN | F | $F^x$ | '-' |
| (47) | 3 | 2 | -CN | F | $F^x$ | '-' |

$^x$Z in the ortho-position to X

**11.** Phenylcyclohexanes according to Claim 1, characterized in that n, r, X, Y, Z and A have the following meanings:

| | n | r | X | Y | Z | A |
|---|---|---|---|---|---|---|
| (57) | 3 | 0 | F | F | H | Cyc |
| (67) | 0 | 0 | Cl | H | H | Cyc |
| (68) | 1 | 0 | Cl | H | H | Cyc |
| (72) | 0 | 0 | Cl | F | H | Cyc |
| (73) | 1 | 0 | Cl | F | H | Cyc |
| (77) | 0 | 0 | $-CF_3$ | H | H | Cyc |
| (78) | 1 | 0 | $-CF_3$ | H | H | Cyc |
| (79) | 0 | 2 | $-CF_3$ | H | H | Cyc |
| (80) | 1 | 2 | $-CF_3$ | H | H | Cyc |
| (81) | 3 | 2 | $-CF_3$ | H | H | Cyc |
| (82) | 0 | 0 | $-OCF_3$ | H | H | Cyc |
| (83) | 1 | 0 | $-OCF_3$ | H | H | Cyc |
| (84) | 0 | 2 | $-OCF_3$ | H | H | Cyc |
| (85) | 1 | 2 | $-OCF_3$ | H | H | Cyc |
| (86) | 3 | 2 | $-OCF_3$ | H | H | Cyc |
| (87) | 0 | 0 | $-OCHF_2$ | H | H | Cyc |
| (88) | 1 | 0 | $-OCHF_2$ | H | H | Cyc |
| (89) | 0 | 2 | $-OCHF_2$ | H | H | Cyc |
| (90) | 1 | 2 | $-OCHF_2$ | H | H | Cyc |
| (91) | 3 | 2 | $-OCHF_2$ | H | H | Cyc |
| (97) | 0 | 0 | -CN | F | $F^x$ | Cyc |
| (98) | 1 | 0 | -CN | F | $F^x$ | Cyc |
| (99) | 0 | 2 | -CN | F | $F^x$ | Cyc |
| (100) | 1 | 2 | -CN | F | $F^x$ | Cyc |
| (101) | 3 | 2 | -CN | F | $F^x$ | Cyc |

$^x$Z in the ortho-position to X

**12.** Phenylcyclohexanes according to Claim 1, characterized in that n, r, X, Y, Z and A have the following meanings:

| | n | r | X | Y | Z | A |
|---|---|---|---|---|---|---|
| (102) | 3 | 0 | F | F | H | Phe |
| (112) | 0 | 0 | Cl | H | H | Phe |
| (113) | 1 | 0 | Cl | H | H | Phe |
| (117) | 0 | 0 | Cl | F | H | Phe |
| (118) | 1 | 0 | Cl | F | H | Phe |
| (122) | 0 | 0 | $-CF_3$ | H | H | Phe |
| (123) | 1 | 0 | $-CF_3$ | H | H | Phe |
| (124) | 0 | 2 | $-CF_3$ | H | H | Phe |
| (125) | 1 | 2 | $-CF_3$ | H | H | Phe |
| (126) | 3 | 2 | $-CF_3$ | H | H | Phe |
| (127) | 0 | 0 | $-OCF_3$ | H | H | Phe |
| (128) | 1 | 0 | $-OCF_3$ | H | H | Phe |
| (129) | 0 | 2 | $-OCF_3$ | H | H | Phe |
| (130) | 1 | 2 | $-OCF_3$ | H | H | Phe |
| (131) | 3 | 2 | $-OCF_3$ | H | H | Phe |
| (132) | 0 | 0 | $-OCHF_2$ | H | H | Phe |
| (133) | 1 | 0 | $-OCHF_2$ | H | H | Phe |
| (134) | 0 | 2 | $-OCHF_2$ | H | H | Phe |
| (135) | 1 | 2 | $-OCHF_2$ | H | H | Phe |
| (136) | 3 | 2 | $-OCHF_2$ | H | H | Phe |
| (137) | 0 | 0 | -CN | F | H | Phe |
| (138) | 1 | 0 | -CN | F | H | Phe |
| (139) | 0 | 2 | -CN | F | H | Phe |
| (140) | 1 | 2 | -CN | F | H | Phe |
| (141) | 3 | 2 | -CN | F | H | Phe |
| (142) | 0 | 0 | -CN | F | $F^x$ | Phe |
| (143) | 1 | 0 | -CN | F | $F^x$ | Phe |
| (144) | 0 | 2 | -CN | F | $F^x$ | Phe |
| (145) | 1 | 2 | -CN | F | $F^x$ | Phe |
| (146) | 3 | 2 | -CN | F | $F^x$ | Phe |

$^x$Z in the ortho-position to X

**13.** Process for the preparation of phenylcyclohexanes according to Claim 2, characterized in that either an aldehyde of the formula II

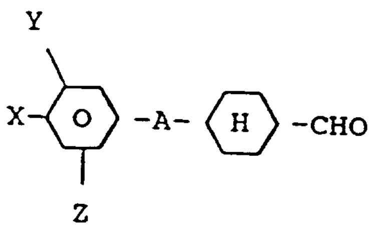

II

is reacted with a phosphonium salt by the Wittig method or a phosphonium salt of the formula III

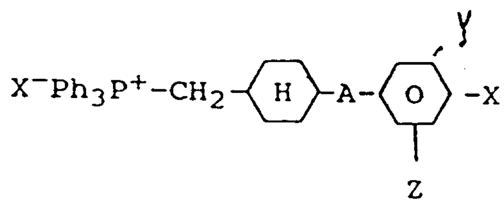

III

is reacted by the Wittig method with a corresponding aldehyde.

**14.** Process for the preparation of phosphonium salts of the formula III according to Claim 13, characterized in that a cyclohexylmethyl alcohol of the formula

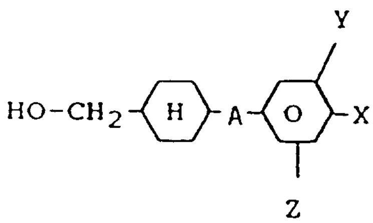

is reacted, after conversion to the cyclohexylmethyl iodide, with triphenylphosphine.

**15.** Cyclohexylmethyl alcohol of the formula

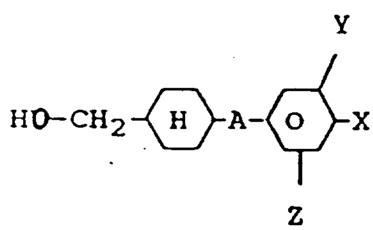

in which Y = F, Z = H or F, X = F, Cl, $-CF_3$, -CN, $-OCF_3$ or $OCHF_2$ and A is trans-1,4-cyclohexylene, 1,4-phenylene, 3-fluoro-1,4-phenylene or a single bond.

**16.** Intermediate of the formula II

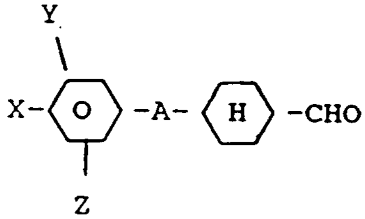

in which Y = F, Z = H or F, X, [sic] F, Cl, $-CF_3$, -CN, $-OCF_3$ or $-OCHF_2$ and A is trans-1,4-cyclohexylene, 1,4-phenylene, 3-fluoro-1,4-phenylene or a single bond.

**17.** Use of the phenylcyclohexanes of the formula I according to one of Claims 2 to 12 [lacuna] components of liquid-crystalline media for electrooptical displays.

**18.** Liquid-crystalline medium for electrooptical displays having at least two liquid-crystalline components, characterized in that at least one component [lacuna] a phenylcyclohexane according to one of Claims 2 to 12.

**19.** Electrooptical display based on a liquid-crystal cell, characterized in that the liquid-crystal cell contains a medium according to Claim 1.

**20.** Electrooptical display based on a liquid-crystal cell, characterized in that the liquid-crystal cell contains a medium according to Claim 18.

**Revendications**

**1.** Milieu à cristaux liquides pour affichages électro-optiques à au moins deux composants à cristaux liquides, caractérisé en ce qu'au moins un composant consiste en un phénylcyclohexane de formule I

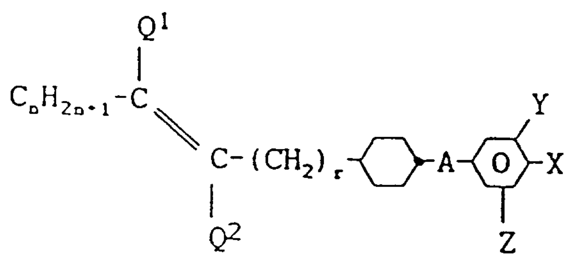

dans laquelle n est un nombre allant de 0 à 7, $Q^1$ et $Q^2$ représentent H, r est égal à 0, 1 ou 2, A représente un cycle trans-1,4-cyclohexylène, 1,4-phénylène, 3-fluoro-1,4-phénylène ou une liaison simple, X représente F, Cl, $-CF_3$, -CN, $-OCF_3$ ou $-OCHF_2$, et Y et Z représentent chacun, indépendamment l'un de l'autre, H ou F, sous réserve que lorsque X = CN, Y et/ou Z représentent F, et en ce qu'il contient, en tant qu'autre constituant, un composé de formule 5

R'-L-C≡C-E-R''    5

dans laquelle L et E, qui peuvent avoir des significations identiques ou différentes, représentent chacun, indépendamment l'un de l'autre, un radical bivalent choisi parmi -Phe-, -Cyc-, -Phe-Phe-, -Phe-Cyc-, -Cyc-Cyc-, -Pyr-, -Dio-, -G-Phe- et G-Cyc- et leurs images spéculaires, Phe représentant un groupe 1,4-phénylène non substitué ou substitué par le fluor, Cyc un groupe trans-1,4-cyclohexylène ou 1,4-cyclohexénylène, Pyr un groupe pyrimidine-2,5-diyle ou pyridine-2,5-diyle, Dio un groupe 1,3-dioxanne-2,5-diyle et G un groupe 2-(trans-1,4-cyclohexyl)-éthyle, pyrimidine-2,5-diyle, pyridine-2,5-diyle ou 1,3-dioxanne-2,5-diyle, et R' et R'' représentent chacun, indépendamment l'un de l'autre, un groupe alkyle, alcényle, alcoxy, alcényloxy ou alcanoyloxy contenant jusqu'à 8 atomes de carbone, R'' pouvant également représenter -CN, $-CF_3$, $-OCF_3$, $-OCHF_2$, F, Cl ou -NCS.

**2.** Phénylcyclohexanes selon la revendication 1, caractérisés en ce que Y = F, Z = H ou F et r = 0.

**3.** Phénylcyclohexanes selon la revendication 2, caractérisés en ce que X représente F, Cl, $-CF_3$, $-OCHF_2$ ou $-OCF_3$.

**4.** Phénylcyclohexanes selon la revendication 1, caractérisés en ce que r = 0 et X = Cl, $-OCF_3$ ou $-OCHF_2$.

**5.** Phénylcyclohexanes selon la revendication 1, caractérisés en ce que

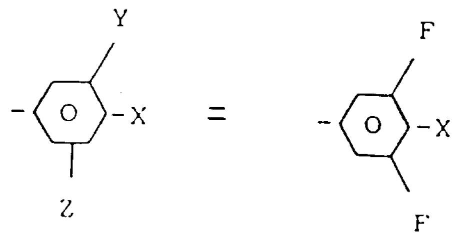

**6.** Phénylcyclohexanes selon l'une des revendications 1, 2, 3, 4 ou 5, caractérisés en ce que X représente $-CF_3$, $-OCF_3$ ou $-OCHF_2$.

**7.** Phénylcyclohexanes selon la revendication 5, caractérisés en ce que X = CN, Z = F et Y = H ou F.

**8.** Phénylcyclohexanes de formule I

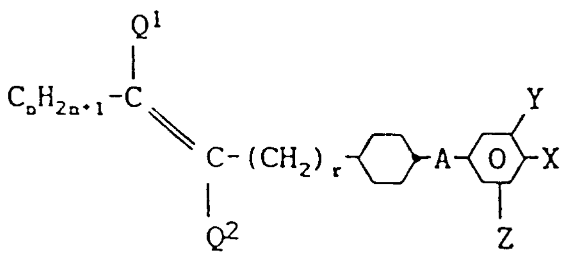

dans laquelle n est un nombre allant de 0 à 7, $Q^1$ et $Q^2$ représentent H, r est égal à 2 ou 3, A représente un groupe 1,4-phénylène, 3-fluoro-1,4-phénylène ou une liaison simple, X représente F ou Cl, Z = H et Y = F ou H.

**9.** Le 4-trans-4-(1-butényl)-cyclohexyl-1,2-difluorobenzène.

**10.** Phénylcyclohexanes selon la revendication 1, caractérisés en ce que n, r, X, Y, Z et A ont les significations suivantes :

| | n | r | X | Y | Z | A |
|---|---|---|---|---|---|---|
| (3) | 3 | 0 | F | F | H | '-' |
| (8) | 0 | 0 | F | H | H | '-' |
| (9) | 1 | 0 | F | H | H | '-' |
| (13) | 0 | 0 | Cl | H | H | '-' |
| (14) | 1 | 0 | Cl | H | H | '-' |
| (18) | 0 | 0 | Cl | F | H | '-' |
| (19) | 1 | 0 | Cl | F | H | '-' |
| (23) | 0 | 0 | $-CF_3$ | H | H | '-' |
| (24) | 1 | 0 | $-CF_3$ | H | H | '-' |
| (25) | 0 | 2 | $-CF_3$ | H | H | '-' |
| (26) | 1 | 2 | $-CF_3$ | H | H | '-' |
| (27) | 3 | 2 | $-CF_3$ | H | H | '-' |
| (28) | 0 | 0 | $-OCF_3$ | H | H | '-' |
| (29) | 1 | 0 | $-OCF_3$ | H | H | '-' |
| (30) | 0 | 2 | $-OCF_3$ | H | H | '-' |
| (31) | 1 | 2 | $-OCF_3$ | H | H | '-' |
| (32) | 3 | 2 | $-OCF_3$ | H | H | '-' |
| (33) | 0 | 0 | $-OCHF_3$ | H | H | '-' |
| (34) | 1 | 2 | $-OCHF_3$ | H | H | '-' |
| (35) | 0 | 2 | $-OCHF_3$ | H | H | '-' |
| (36) | 1 | 2 | $-OCHF_3$ | H | H | '-' |
| (37) | 3 | 2 | $-OCHF_3$ | H | H | '-' |
| (43) | 0 | 0 | -CN | F | $F^x$ | '-' |
| (44) | 1 | 0 | -CN | F | $F^x$ | '-' |
| (45) | 0 | 2 | -CN | F | $F^x$ | '-' |
| (46) | 1 | 2 | -CN | F | $F^x$ | '-' |
| (47) | 3 | 2 | -CN | F | $F^x$ | '-' |

*) Z est en position ortho par rapport à X.

**11.** Phénylcyclohexanes selon la revendication 1, caractérisés en ce que n, r, X, Y, Z et A ont les significations suivantes :

| | n | r | X | Y | Z | A |
|---|---|---|---|---|---|---|
| (57) | 3 | 0 | F | F | H | Cyc |
| (67) | 0 | 0 | Cl | H | H | Cyc |
| (68) | 1 | 0 | Cl | H | H | Cyc |
| (72) | 0 | 0 | Cl | F | H | Cyc |
| (73) | 1 | 0 | Cl | F | H | Cyc |
| (77) | 0 | 0 | $-CF_3$ | H | H | Cyc |
| (78) | 1 | 0 | $-CF_3$ | H | H | Cyc |
| (79) | 0 | 2 | $-CF_3$ | H | H | Cyc |
| (80) | 1 | 2 | $-CF_3$ | H | H | Cyc |
| (81) | 3 | 2 | $-CF_3$ | H | H | Cyc |
| (82) | 0 | 0 | $-OCF_3$ | H | H | Cyc |
| (83) | 1 | 0 | $-OCF_3$ | H | H | Cyc |
| (84) | 0 | 2 | $-OCF_3$ | H | H | Cyc |
| (85) | 1 | 2 | $-OCF_3$ | H | H | Cyc |
| (86) | 3 | 2 | $-OCF_3$ | H | H | Cyc |
| (87) | 0 | 0 | $-OCHF_2$ | H | H | Cyc |
| (88) | 1 | 0 | $-OCHF_2$ | H | H | Cyc |
| (89) | 0 | 2 | $-OCHF_2$ | H | H | Cyc |
| (90) | 1 | 2 | $-OCHF_2$ | H | H | Cyc |
| (91) | 3 | 2 | $-OCHF_2$ | H | H | Cyc |
| (97) | 0 | 0 | -CN | F | $F^x$ | Cyc |
| (98) | 1 | 0 | -CN | F | $F^x$ | Cyc |
| (99) | 0 | 2 | -CN | F | $F^x$ | Cyc |
| (100) | 1 | 2 | -CN | F | $F^x$ | Cyc |
| (101) | 3 | 2 | -CN | F | $F^x$ | Cyc |

*) Z est en position ortho par rapport à X.

**12.** Phénylcyclohexanes selon la revendication 1, caractérisés en ce que n, r, X, Y, Z et A ont les significations suivantes :

| | n | r | X | Y | Z | A |
|---|---|---|---|---|---|---|
| (102) | 3 | 0 | F | F | H | Phe |
| (112) | 0 | 0 | Cl | H | H | Phe |
| (113) | 1 | 0 | Cl | H | H | Phe |
| (117) | 0 | 0 | Cl | F | H | Phe |
| (118) | 1 | 0 | Cl | F | H | Phe |
| (122) | 0 | 0 | $-CF_3$ | H | H | Phe |
| (123) | 1 | 0 | $-CF_3$ | H | H | Phe |
| (124) | 0 | 2 | $-CF_3$ | H | H | Phe |
| (125) | 1 | 2 | $-CF_3$ | H | H | Phe |
| (126) | 3 | 2 | $-CF_3$ | H | H | Phe |
| (127) | 0 | 0 | $-OCF_3$ | H | H | Phe |
| (128) | 1 | 0 | $-OCF_3$ | H | H | Phe |
| (129) | 0 | 2 | $-OCF_3$ | H | H | Phe |
| (130) | 1 | 2 | $-OCF_3$ | H | H | Phe |
| (131) | 3 | 2 | $-OCF_3$ | H | H | Phe |
| (132) | 0 | 0 | $-OCHF_2$ | H | H | Phe |
| (133) | 1 | 0 | $-OCHF_2$ | H | H | Phe |
| (134) | 0 | 2 | $-OCHF_2$ | H | H | Phe |
| (135) | 1 | 2 | $-OCHF_2$ | H | H | Phe |
| (136) | 3 | 2 | $-OCHF_2$ | H | H | Phe |
| (137) | 0 | 0 | -CN | F | H | Phe |
| (138) | 1 | 0 | -CN | F | H | Phe |
| (139) | 0 | 2 | -CN | F | H | Phe |
| (140) | 1 | 2 | -CN | F | H | Phe |
| (141) | 3 | 2 | -CN | F | H | Phe |
| (142) | 0 | 0 | -CN | F | $F^x$ | Phe |
| (143) | 1 | 0 | -CN | F | $F^x$ | Phe |
| (144) | 0 | 2 | -CN | F | $F^x$ | Phe |
| (145) | 1 | 2 | -CN | F | $F^x$ | Phe |
| (146) | 3 | 2 | -CN | F | $F^x$ | Phe |

*) Z est en position ortho par rapport à X.

**13.** Procédé de préparation des phénylcyclohexanes selon la revendication 2, caractérisé en ce que l'on fait réagir un aldéhyde de formule II

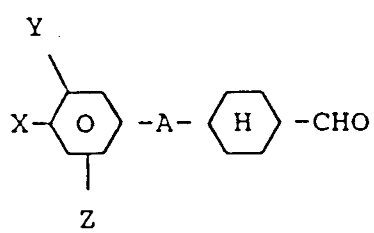

II

avec un sel de phosphonium, selon Wittig,
ou bien en ce que l'on fait réagir un sel de phosphonium de formule III

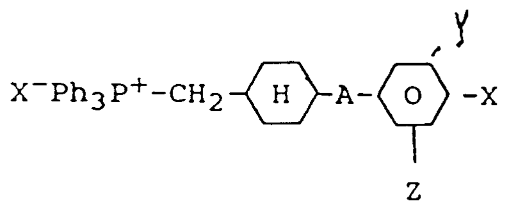

III

avec un aldéhyde correspondant selon Wittig.

**14.** Procédé de préparation des sels de phosphonium de formule III de la revendication 13, caractérisé en ce que l'on fait réagir un alcool cyclohexylméthylique de formule

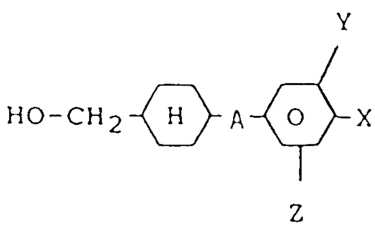

après conversion en l'iodure de cyclohexylméthyle, avec la triphénylphosphine.

**15.** Alcool cyclohexylméthylique de formule

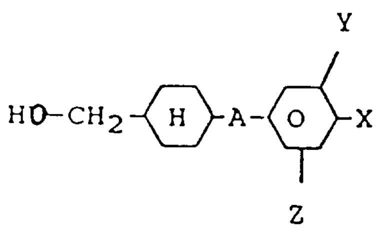

dans laquelle Y = F, Z = H ou F, X = F, Cl, $-CF_3$, -CN, $-OCF_3$ ou $-OCHF_2$ et A représente un groupe trans-1,4-cyclohexylène, 1,4-phénylène, 3-fluoro-1,4-phénylène ou une liaison simple.

**16.** Produit intermédiaire de formule II

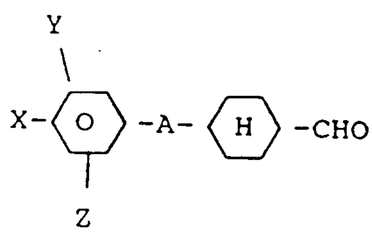

dans laquelle Y = F, Z = H ou F, X représente F, Cl, $-CF_3$, -CN, $-OCF_3$ ou $OCHF_2$ et A représente un groupe trans-1,4-cyclohexylène, 1,4-phénylène, 3-fluoro-1,4-phénylène ou une liaison simple.

**17.** Utilisation des phénylcyclohexanes de formule I selon l'une des revendications 2 à 12 en tant que composants de milieux à cristaux liquides pour affichages électro-optiques.

**18.** Milieu à cristaux liquides pour affichages électro-optiques, à au moins deux composants à cristaux liquides, caractérisé en ce qu'un composant au moins consiste en un phénylcyclohexane selon l'une

des revendications 2 à 12.

**19.** Affichages électro-optiques à base d'une cellule à cristaux liquides, caractérisés en ce que la cellule à cristaux liquides contient un milieu selon la revendication 1.

**20.** Affichages électro-optiques à base d'une cellule à cristaux liquides, caractérisés en ce que la cellule à cristaux liquides contient un milieu selon la revendication 18.